# EUROPEAN PATENT APPLICATION

(11) **EP 4 588 451 A1**
(43) Date of publication of application: **23.07.2025**
(21) Application number: 23864141.9
(22) Date of filing: 11.09.2023
(51) Int. Cl.: A61B 34/30, A61B 17/00

(54) **MOVABLE LONG STRUCTURE AND METHOD FOR MANIPULATING SAME, MEDICAL SYSTEM, TOOL, ROBOT AND METHOD FOR OPERATING SAME, MANIPULATOR, FLEXIBLE ENDOSCOPE, AND STEERING CATHETER**

(30) Priority: 12.09.2022 US 202263405512 P
(71) Applicant: National University Corporation Shiga University of Medical Science, Otsu-shi Shiga 520-2192 (JP)
(72) Inventor: YAMADA, Atsushi, Otsu-shi, Shiga 520-2192 (JP); TANI, Tohru, Otsu-shi, Shiga 520-2192 (JP)
(74) Representative: Gunzelmann, Rainer
(86) International application number: PCT/JP2023/032989
(87) International publication number: WO 2024/058098

(57) **Abstract**

[summary] An object of the present invention is to provide a movable elongated structure etc., in which at least the distal end side may be curved and deformed in a desired direction with a simple structure.

[Solution] Eight traction wires 50 are fastened to fixed recesses 61 of a distal end side cap 60 by enlarged diameter portions 51, wherein in the distal end side flexible tube 20, the eight traction wires 50 are arranged in the distal end side upper lumen set 23 in the upward direction HU among the four directions (HU, HD, WR, WL) of the cross section in which lumen sets 23, 24, 25 and 26 are provided, and wherein in the base end side flexible tube 30, the eight traction wires are arranged in the base end side wire lumens 35b and 36a which are closer to the cross-sectional center "0" in the upward direction HU than the distal end side upper lumen set 23 and have a wider interval between the arranged traction wires 53a and 53b. Therefore, by towing the traction wires 53a and 55b toward the base end side flexible tube 30, the distal end side flexible tube 20 may be bent and deformed in a desired direction relative to the base end side flexible tube 30.

## Description

### TECHNICAL FIELD

The present invention relates, for example, to a movable elongated structure that is inserted into a duct such as a hollow organ, a vessel, or a blood vessel, and has its distal end portion bent and deformed in a predetermined direction at a branching point, etc., and in particular to a movable elongated structure, a movable elongated treatment instrument, a method for inserting a movable elongated structure, a method for operating a movable elongated structure, a movable elongated structural instrument, a medical system, a tool, a robot, a method for operating a robot, a medical robot, a manipulator, a flexible endoscope, and a steering catheter.

### BACKGROUND OF THE INVENTION

For example, long medical instruments that are inserted into body cavities, such as catheters and endoscopes, are widely used. Such long medical instruments have their distal end portions bent and inserted into branched portions, as in the medical manipulator described in Patent Document 1.

The endoscope provided on the manipulator of Patent Document 1 is configured such that a bending portion provided in the middle of its distal end side may be bent in four directions, up, down, left, and right, thereby changing the direction of the distal end. However, the structure is complicated.

### PRIOR ART DOCUMENTS

### [Patent documents]

[Patent Document 1] Japanese Patent Application Laid-Open No. Hei 8-224247

### SUMMARY OF THE INVENTION

### [Problem to be solved by the invention]

Therefore, the present invention aims to provide a movable elongated structure, a movable elongated treatment instrument, a method for inserting a movable elongated structure, a method for operating a movable elongated structure, a movable elongated structure instrument, a medical system, a tool, a robot, a method for operating a robot, a medical robot, a manipulator, a flexible endoscope, and a steering catheter, which are capable of bending and deforming at least the distal end side in a desired direction with a simple structure,

### [Means for solving the problem]

The present invention provides a movable elongated structure which includes a plurality of tubular (or cylindrical) bodies formed in a flexible, elongated shape and having an internal space penetrating therein in the longitudinal direction, a flexible, elongated or long traction (or towing) body, and a or wiring aid (or wiring aid tool) arranged between two of the plurality of tubular bodies arranged in series and adjacent to each other in the longitudinal direction, and maintaining a distance between the opposing ends of the tubular bodies, wherein the direction in which the traction body is towed in the longitudinal direction is the base end side and the opposite side is the distal end side, and among the two tubular bodies adjacent to each other in the longitudinal direction in the plurality of tubular bodies arranged in series, the tubular body arranged on the base end side is a base end side tubular body and the tubular body arranged on the distal end side is a distal end side tubular body, a wire lumen is provided in the tubular bodies for wiring the traction body along the longitudinal direction, and the traction body is wired in the wiring aid at an angle (obliquely) with respect to the longitudinal direction, thereby allowing at least the distal end side tubular body to be curved and deformed in a desired direction.

A cylindrical body may be employed as a tubular body such as a flexible tube, or a flexible cylindrical body formed by a so-called skeletal structure in which a plurality of cylindrical bodies is connected in a bendable manner.

The present invention also provides a movable elongated structure which includes a plurality of tubular bodies formed in a flexible, elongated shape and having an internal space penetrating therein in the longitudinal direction, a flexible, elongated traction body, and a wiring aid arranged between two of the plurality of tubular bodies arranged in series and adjacent to each other in the longitudinal direction, and maintaining a distance between the opposing ends of the tubular bodies,
wherein the direction in which the traction body is towed in the longitudinal direction is the base end side and the opposite side is the distal end side,
wherein among the two tubular bodies adjacent in the longitudinal direction in the plurality of tubular bodies arranged in series, the tubular body arranged on the base end side is referred to as a base end side tubular body, and the tubular body arranged on the distal end side is referred to as a distal end side tubular body,
wherein a pair of the traction bodies is referred to as a traction body set, and four sets of the traction body sets are provided, and a wire lumen for wiring the traction bodies along the longitudinal direction is provided in the tubular body, and the pair of the wire lumens is referred to as a wire lumen set, and four sets of the wire lumen sets are provided in which four sets of the wire lumen sets are arranged in four directions in the cross section of the tubular bodies,
wherein the base end side tubular body and the distal end side tubular body arranged in series are arranged so that the wire lumen set provided in the four directions is oriented along the longitudinal direction,
wherein the traction body set is arranged in the distal end side tubular body in one of the four directions of the cross section in which the wire lumen sets are provided,
wherein in the base end side tubular body, the cross-sectional center of the tubular body in the one direction is closer to the wire lumen set arranged in the distal end side tubular body than the wire lumen set arranged in the distal end side tubular body, and the traction bodies are arranged in the wire lumen in which the distance between the traction bodies is wider,
wherein in the distal end side tubular body, the two traction body sets are arranged in the two wire lumen sets that face each other in one direction across the cross-sectional center of the tubular body,
wherein in the base end side tubular body, the traction bodies in the two traction body sets are arranged in one of the wire lumens in the two wire lumen sets that face each other across the cross-sectional center of the tubular body in a cross direction that crosses the one direction, and wherein in the base end side tubular body, a first virtual line connecting the traction bodies of one of the two traction body sets arranged in one of the wire lumens in the two wire lumen sets in the cross-sectional direction and a second virtual line connecting the traction bodies of the other traction body set in the cross-sectional direction intersect in the cross-sectional direction, and by towing the multiple traction bodies toward the base end side, at least one of the base end side tubular body and the distal end side tubular body is curved and deformed in a desired direction relative to the other.

The present invention also provides a movable elongated structure which includes a plurality of tubular bodies formed in a flexible, elongated shape and having an internal space passing through in the longitudinal direction, a flexible, elongated traction body; and a wiring aid arranged between two of the plurality of tubular bodies arranged in series and adjacent to each other in the longitudinal direction, and maintaining a distance between opposing ends of the tubular bodies, wherein a direction in which the traction body is towed in the longitudinal direction is defined as a base end side and an opposite side is defined as a distal end side, and in the two tubular bodies adjacent to each other in the longitudinal direction among the plurality of tubular bodies arranged in series, the tubular body arranged on the base end side is defined as a base end side tubular body and the tubular body arranged on the distal end side is defined as a distal end side tubular body, wherein a pair of traction bodies is a traction body set, and two sets of traction body sets are provided, a wire lumen for wiring the traction bodies along the longitudinal direction is provided in the tubular bodies, the pair of wire lumens is a wire lumen set, two sets of wire lumen sets are provided in the tubular body, the two sets of wire lumen sets are arranged at opposing positions in the cross section of the tubular body, wherein the distal end side tubular body is arranged in series with the base end tubular body in such a direction that the opposing directions in which the wire lumens in the distal end side tubular body are arranged cross the opposing directions in which the wire lumen sets in the base end side tubular body are arranged, wherein two sets of traction body sets are arranged in the distal end side tubular body to the two sets of wire lumen sets, wherein in the base end side tubular body, the traction body of one of the two sets of traction body sets is respectively arranged to either one of the wire lumen sets, wherein the traction body of the other traction body set is respectively arranged in the unrouted wire lumens of the two wire lumen sets, wherein in the base-end side tubular body, the traction bodies of the two traction body sets are arranged in either of the wire lumens of the two wire lumen sets that face each other across the cross-sectional center of the tubular body in a crossing direction that crosses one direction, wherein in the base end side tubular body, a first imaginary line connecting the traction bodies of one of the traction body sets of the two traction body sets arranged in any of the wire lumens in the two wire lumen sets in the cross-sectional direction and a second imaginary line connecting the traction bodies of the other traction body set in the cross-sectional direction intersect in the cross-sectional direction, and wherein by towing the multiple traction bodies toward the base end side, at least one of the base end side tubular body and the distal end side tubular body is curved and deformed in a desired direction. relative to the other.

The present invention also provides a movable elongated structure including a plurality of tubular bodies formed in a flexible, long shape and having an internal space passing through in the longitudinal direction; a long, flexible traction body; and a wiring aid arranged between two of the tubular bodies adjacent in the longitudinal direction among the plurality of tubular bodies arranged in series and maintaining a distance between opposing ends of the tubular bodies, wherein the direction in which the traction body is towed in the longitudinal direction is the base end side and the opposite side is the distal end side, and in the two tubular bodies adjacent in the longitudinal direction among the plurality of tubular bodies arranged in series, the tubular body arranged on the base end side is referred to as a base end side tubular body and the tubular body arranged on the distal end side is referred to as a distal end side tubular body, wherein a pair of the traction bodies is a traction body set, and four sets of the traction body sets are provided, a wire lumen for wiring the traction body along the longitudinal direction is provided in the tubular bodies, the pair of the wire lumens are referred to as a wire lumen set, and four sets of the wire lumen sets are provided, and the four sets of the wire lumen sets are arranged in four directions in a cross section of the tubular body, wherein the wire lumen sets arranged in four directions are arranged in the longitudinal direction of the base end side tubular body and the distal end side tubular body, wherein the traction body set is arranged in the wire lumen set in one of the four directions of the cross section in which the wire lumen set is provided in the distal end side tubular body, and arranged in the wire lumen in the base end side tubular body, which is closer to the cross-sectional center of the tubular body in the one direction than the wire lumen set arranged in the distal end side tubular body and has a wider interval between the traction bodies arranged, wherein the traction bodies are arranged in the wire lumen of the base end side tubular body and the wire lumen of the distal end side tubular body, and are configured to be independently towed by being fastened to the distal end side tubular body at the distal end side of the distal end side tubular body, and wherein at least one of the base end side tubular body and the distal end side tubular body is curved and deformed in a desired direction relative to the other by towing the multiple traction bodies toward the base end side.

The present invention also provides a movable elongated structure including a plurality of tubular bodies formed in a flexible, long shape and having an internal space passing through in the longitudinal direction; a long, flexible traction body; and a wiring aid arranged between two of the tubular bodies adjacent in the longitudinal direction among the plurality of tubular bodies arranged in series and maintaining a distance between opposing ends of the tubular bodies, wherein the direction in which the traction body is towed in the longitudinal direction is the base end side and the opposite side is the distal end side, and in the two tubular bodies adjacent in the longitudinal direction among the plurality of tubular bodies arranged in series, the tubular body arranged on the base end side is referred to as a base end side tubular body and the tubular body arranged on the distal end side is referred to as a distal end side tubular body, wherein pair of the traction bodies is a traction body set, and four sets of the traction body sets are provided, wherein one of the pair of traction bodies is a long traction body that is routed across the base end side tubular body and the distal end side tubular body, and the other is a short traction body that is routed to the base end side tubular body, wherein a pair of the wire lumens is a wire lumen set, and four sets of the wire lumen sets are provided, wherein the four wire lumen sets are arranged in four directions in the cross section of the base end side tubular body, wherein the four wire lumens are arranged in four directions in the cross section of the distal end side tubular body, and arranged with the base end side tubular body and the distal end side tubular body that are arranged in series in a direction that the four directions are aligned, wherein the long traction body of the traction body set is routed to the wire lumen in one of the four directions of the cross section in which the wire lumen is provided in the distal end side tubular body, and fixed to the distal end side tubular body at the distal end of the distal end side tubular body, wherein the long and short traction bodies of the traction body set are arranged in the base end side tubular body in the wire lumen that is closer to the cross-sectional center of the tubular body in the one direction than the wire lumen arranged in the distal end side tubular body and faces the cross-sectional center in the one direction, wherein the short traction body arranged in the wire lumen of the base end side tubular body is fixed to the base end side tubular body on the distal side of the base end side tubular body, wherein each of the traction bodies is configured to be independently towable, and wherein by towing the plurality of traction bodies toward the base end side, at least one of the base end side tubular body and the distal end side tubular body is curved and deformed in a desired direction relative to the other.

Furthermore, the present invention provides A movable elongated structure including a plurality of tubular bodies formed in a flexible, long shape and having an internal space passing through in the longitudinal direction, a flexible, long-shaped traction body, a wiring aid arranged between two of the tubular bodies arranged in series in the longitudinal direction and maintaining a distance between the opposing ends of the tubular bodies, wherein the direction in which the traction body is towed in the longitudinal direction is the base end side and the opposite side is the distal end side, wherein in the two tubular bodies adjacent in the longitudinal direction among the plurality of tubular bodies arranged in series, the tubular body arranged on the base end side is the base end side tubular body and the tubular body arranged on the distal end side is the distal end side tubular body, wherein a wire lumen is provided in the tubular bodies for wiring the traction body along the longitudinal direction, wherein a pair of the traction bodies is a traction body set, and a pair of the wire lumens is a wire lumens set, wherein in the base end side tubular body, the wire lumens are arranged in a wire lumen that is closer to the cross-sectional center of the tubular body in the one direction and has a wider interval between the wire lumens than the wire lumen set arranged in the distal end side tubular body, wherein in the base end side tubular body, a first virtual line connecting the traction bodies of one of the two sets of the traction body sets arranged in one of the wire lumens in the two sets of the wire lumen sets in the cross-sectional direction and a second virtual line connecting the traction bodies of the other set of the traction body in the cross-sectional direction intersect in the cross-sectional direction, wherein by towing the plurality of traction bodies toward the base end side, at least one of the base end side tubular body and the distal end tubular body is curved and deformed in a desired direction relative to the other.

As an aspect of the present invention, the first virtual line and the second virtual line may pass through a center of the cross section.

As another aspect of the present invention, the wiring aid may be provided with a regulating portion that regulates the relative positions of the traction body of one of the two traction body sets that cross between the tubular bodies adjacent to each other in the longitudinal direction and the traction body of the other traction body set.

As a further aspect of the present invention, the traction body may be a flexible wire, and the wire lumen may be a through passage provided inside a tube wall of the tubular body along the longitudinal direction.

The present invention may also be a movable elongated treatment instrument in which a treatment instrument such as a retractor, gripper, forceps, tweezers, needle, probe, or scissors is provided at the distal end of the distal tubular body in the movable elongated structure, and a drive mechanism for the treatment instrument is disposed in the internal space. Note that a cable for transmitting high frequency (radio wave) or microwave may be wired to the treatment instrument in order to heat, cauterize, stop bleeding, or cut biological tissue with the treatment instrument.

As another aspect of the present invention, the distal end tubular body may be an elastic retractor, an elastic gripper, a forceps or a scissors having the internal space, and the wire may be used to open and close the distal end tubular body.

The present invention also provides a method for inserting a movable elongated structure, which comprises inserting the above-mentioned movable elongated structure into a pipeline, driving and controlling a traction drive unit that traction body to bend and deform at least one of the distal end side tubular body and the base end side tubular body in a desired direction, and inserting the distal end side tubular body into a branching pipeline.

In a further aspect of the present invention, the duct may be at least one of a hollow organ, a vascular tract, and a blood vessel.

The present invention also provides a method for operating a movable elongated structure, in which the traction body set is towed to bend and deform at least one of the distal end side tubular body and the base end side tubular body in a desired direction in the above-mentioned movable elongated structure.

The present invention also provides in the above-mentioned movable elongated structure, a method for operating a movable elongated structure in which one of the plurality of sets of traction bodies is towed, thereby bending and deforming at least one of the distal end side tubular body and the base end side tubular body in a desired direction.

The present invention also provides a method for operating a movable elongated structure including wiring the traction body set to the wire lumen of the base end tubular body and the wire lumen of the distal end tubular body, towing one of the traction bodies attached to the distal end tubular body at the distal end side of the distal end tubular body to bend and deform the distal end tubular body in a desired direction, wiring the traction body set to the wire lumen of the base end tubular body, and towing the other of the traction body set attached to the base end tubular body at the distal end side of the base end tubular body to bend and deform the base end tubular body in a desired direction.

The present invention also provides a movable elongated structure instrument including the above-mentioned movable elongated structure and a traction drive unit that traction body, wherein the traction drive unit tows the traction body to bend and deform at least one of the distal end side tubular body and the base end tubular body in a desired direction.

The present invention also provides a medical system including the above-mentioned movable elongated structural instrument, a drive unit for driving the traction drive section, and a control unit connected to apply a drive signal to the drive unit.

The present invention also provides a tool including the above-mentioned movable elongated structural instrument, an attachment portion for attaching the base end side tubular body of the movable elongated structural body to the distal end of a robot arm, and a connection portion for connecting to a drive mechanism on the robot arm side that drives the traction drive portion.

The present invention also provides a robot having the above-mentioned tool, a robot arm having the tool at its distal end, a drive unit that drives the traction drive unit and the robot arm, and a control unit connected to apply a drive signal to the drive unit.

The present invention also provides a robot which includes an input/output unit connected to the above-mentioned movable elongated structural instrument by wire and/or wireless means, an input unit that receives operation signals in real time, an arithmetic unit that executes a predetermined operation program based on the operation signals, and an output unit that generates a drive signal for traction a predetermined traction body using the traction drive unit based on the output from the calculation unit, thereby bending and deforming at least one of the distal end side tubular body and the base end side tubular body in a desired direction.

The present invention also provides comprises a robot which includes the above-mentioned tool, a robot arm having the tool at its distal end, a drive unit that drives the traction drive unit and the robot arm, and a control unit connected to apply a drive signal to the drive unit, wherein the control unit is a robot equipped with artificial intelligence.

The present invention also provides a robot which includes the above-mentioned manipulator, a robot arm having the tool at its distal end, a drive unit that drives the operating unit and the robot arm, and a control unit connected to apply a drive signal to the drive unit, wherein the control unit is a robot equipped with artificial intelligence.

The present invention also provides a robot which includes an input/output unit connected to the above-mentioned movable elongated structural instrument by wire and/or wireless means, an input unit that receives operation signals in real time, an arithmetic unit that executes a predetermined operation program based on the operation signals, and an output unit that generates a drive signal for traction a predetermined traction body using the traction drive unit based on the output from the arithmetic unit, thereby bending and deforming at least one of the distal end side tubular body and the base end side tubular body in a desired direction, wherein the arithmetic unit is a equipped with artificial intelligence.

The present invention also provides a method for operating a robot, in which an input/output unit connected by wire and/or wirelessly to a robot equipped with the above-mentioned movable elongated structural instrument receives operation signals in real time, and a arithmetic unit executes a predetermined operation program based on the received operation signals, and then, based on the output from the arithmetic unit, the traction body is towed by the traction drive unit to bend and deform at least one of the distal end side tubular body and the base end side tubular body in a desired direction.

The present invention also provides a medical robot including the above-mentioned robot, wherein the output unit provides a drive signal to an external drive unit that mechanically drives the movable elongated structure.

The present invention also provides a manipulator including the above-mentioned movable elongated structural instrument, a main body portion provided at the base end of the base end side tubular body in the movable elongated structural body, and an operating portion in the main body portion for operating the traction drive portion.

The present invention also provides a flexible endoscope including the above-mentioned movable elongated structure and a plurality of traction operating units which tow the traction body, and which traction-operating units traction the traction body to bend and deform at least one of the distal end side tubular body and the base end tubular body in a desired direction.

The present invention also provides a steering catheter comprising the above-mentioned movable elongated structure and a plurality of traction operating units which tows the traction body, and which tow the traction body with the traction operating units to curve and deform at least one of the distal end side tubular body and the base end side tubular body in a desired direction.

### EFFECT OF THE INVENTION

According to the present invention, it is possible to provide a movable elongated structure, a movable elongated treatment instrument, a method for inserting a movable elongated structure, a method for operating a movable elongated structure, a movable elongated structure instrument, a medical system, a tool, a robot, a method for operating a robot, a medical robot, a manipulator, a flexible endoscope and a steering catheter, which may bend and deform at least the distal end side in a desired direction with a simple structure by towing a plurality of the traction bodies toward the base end side.

### BRIEF DESCRIPTION OF THE DRAWINGS

FIGS. 1A and 1B are explanatory diagrams of a movable elongated structure. FIG. 1A is a perspective view showing the front, right side, and top of the movable elongated structure, and FIG. 1B is a perspective view showing the front, right side, and top of the movable elongated structure with a distal end side flexible tube, an intermediate tube, and a base end side flexible tube in a see-through state.
FIGS. 2A to 2D are explanatory diagrams of a movable elongated structure. FIG. 2A is a front view of the movable elongated structure, FIG. 2B is a rear view of the movable elongated structure, FIG. 2C is a plan view of the movable elongated structure, and FIG. 2D is a bottom view of the movable elongated structure.
FIGS. 3A to 3D are explanatory diagrams of a spacer (or wiring aid). 3A is a perspective view showing the front, right side and top of the spacer. FIG. 3B is a perspective view showing the front, left side and bottom of the spacer. FIG. 3C is a front view of the spacer, and FIG. 3D is a cross-sectional view taken along the line A-A in FIG. 3C.
FIGS. 4A and 4B are explanatory diagrams of a movable elongated structure. 4A and 4B are explanatory diagrams of a movable elongated structure. Fig. 4A is a development view of the movable elongated structure, and Fig. 4B is a cross-sectional view taken along line B-B in Fig. 2C.
FIGS. 5A and 5B are explanatory diagrams of a movable elongated structure. Fig. 5A is a cross-sectional view taken along line C-C in Fig. 4B, and Fig. 5B is a cross-sectional view taken along line D-D in Fig. 4B.
FIG. 6 is a perspective view showing the front, right side, and top of the movable elongated structure with the distal end side flexible tube bent.
FIGS. 7A and 7B are explanatory diagrams of a movable elongated structure in another embodiment. FIG. 7A is a perspective view showing the front, right side, and top of the movable elongated structure, and FIG 7B is a perspective view showing the front, right side, and top of the movable elongated structure with the distal end side flexible tube, intermediate tube, and base end side flexible tube in a see-through state.
FIGS. 8A to 8D are explanatory diagrams of a movable elongated structure in another embodiment. Fig. 8A is a front view of the movable elongated structure, Fig. 8B is a rear view of the movable elongated structure, Fig. 8C is a plan view of the movable elongated structure, and Fig. 8D is a bottom view of the movable elongated structure.
FIGS. 9A to 9D are explanatory diagrams of a spacer (or wiring aid).in another embodiment. FIG. 9A is a perspective view showing the front, right side and top of the spacer, FIG. 9B is a perspective view showing the front, left side and bottom of the spacer, FIG. 9C is a front view of the spacer, and FIG. 9D is a cross-sectional view taken along the line E-E of FIG. 9C.
FIGS.10A and 10B are explanatory diagrams of a movable elongated structure in another embodiment, in which Fig. 10A is a development view of the movable elongated structure, and Fig. 10B is a cross-sectional view taken along the line F-F in Fig. 8C.
FIGS.11A and 11B are explanatory diagrams of a movable elongated structure in another embodiment. Fig. 11A is a cross-sectional view taken along line G-G in Fig. 10B, and Fig. 11B is a cross-sectional view taken along line H-H in Fig. 10B.
FIG. 12A is a perspective views showing the front, right side and plan views of a movable elongated structure in a state in which a distal end side flexible tube is bent in another embodiment.
FIGS.13A and 13B are explanatory diagrams of a movable elongated structure in still another embodiment. FIG. 13A is a perspective view showing the front, right side, and top of the movable elongated structure, and FIG 13B is a perspective view showing the front, right side, and top of the movable elongated structure with the distal end side flexible tube, intermediate tube, and base end side flexible tube in a see-through state.
FIGS. 14A to 14D are explanatory diagrams of a movable elongated structure in yet another embodiment. Fig. 14A is a front view of the movable elongated structure, Fig. 14B is a rear view of the movable elongated structure, Fig. 14C is a plan view of the movable elongated structure, and Fig. 14D is a bottom view of the movable elongated structure.
FIGS. 15A to 15D are explanatory diagrams of a spacer (or wiring aid) in yet another embodiment. FIG. 15A is a perspective view showing the front, right side and top of the spacer, FIG. 15B is a perspective view showing the front, left side and top of the spacer, FIG. 15C is a front view of the spacer, and FIG. 15D is a cross-sectional view taken along the line I-I in FIG. 15C.
FIGS. 16A and 16B are explanatory diagrams of a movable elongated structure in still another embodiment. Fig. 16A is a development view of the movable elongated structure, and Fig. 16B is a cross-sectional view taken along the line J-J in Fig. 14C.
FIGS. 17A and 17B are explanatory views of a movable elongated structure in still another embodiment. Fig. 17A is a cross-sectional view taken along the line K-K in Fig. 16B, and Fig. 17B is a cross-sectional view taken along the line M-M in Fig. 16B.
FIG. 18 is a perspective view showing the front, right side and top of a movable elongated structure in a state in which a distal end side flexible tube is bent in yet another embodiment.
FIGS. 19A and 19B are schematic explanatory diagrams of a retractor according to another embodiment. FIG. 19A is a perspective view showing the front, right side, and top of the retractor, and FIG 19B is a perspective view showing the front, right side, and top of the retractor with the traction wire shown in a see-through state.
FIGS. 20A to 20C are schematic explanatory diagrams of a retractor according to another embodiment. FIG. 20A is a plan view of the retractor, FiG. 20B is a cross-sectional view of a cross section passing through the upper wire lumen, and FIG. 20C is a plan view of the retractor with the elastic retractor in an open state .
FIG. 21 is a schematic diagram of a medical device according to another embodiment.
FIG. 22 is a schematic diagram of a remote surgical system according to another embodiment.
FIGS. 23A and 23B are schematic diagrams illustrating a surgical device in a remote surgical system, in which FIG. 23A is a plan view of a surgical device that may be mounted on a robot arm assembly of the remote surgical system, and FIG. 23B is a diagram showing the internal configuration of the surgical device in FIG. 23A.
FIGS. 24A and 24B are diagrams illustrating a surgical device in a remote surgical system. FIG. 24A is a block diagram showing the connection relationship between each unit, and FIG. 24B is an operation flow diagram of the remote surgery system.
FIGS. 25A to 25C are front views of the movable elongated structure according to another embodiment of the present invention.

### DETAILED DESCRIPTION OF THE PREFERRED EMBODIMENTS

Hereinafter, an embodiment of the present invention will be described with reference to the drawings. In the following description, the same parts and components are denoted by the same reference numerals. The present embodiment includes, for example, the following disclosure.

### [Configuration 1]

A movable elongated structure (10) includes a plurality of tubular bodies (20 and 30) formed in a flexible long shape and having an internal space (21 and 31) penetrating therein in the longitudinal direction (L), a flexible, long-shaped traction body (50), and a wiring aid (40) arranged between two tubular bodies (20 and 30) adjacent in the longitudinal direction (L) among the plurality of tubular bodies (20 and 30) arranged in series and maintaining a distance between opposing ends of the tubular bodies (20 and 30), the direction in which the traction body (50) is towed in the longitudinal direction (L) being the base end side (LB) and the opposite side being the distal end side (LF), and the plurality of tubular bodies (20) arranged in series In a movable elongated structure (10), of two tubular bodies (20 and 30) adjacent to each other in the longitudinal direction (L) in a wiring aid (40), the tubular body (20 and 30) arranged on the base end side (LB) is designated as the base end side tubular body (30) and the tubular body (20 and 30) arranged on the distal end side (LF) is designated as the distal end side tubular body (20), the tubular bodies (20 and 30) are provided with wire lumens (22 and 32) for wiring a traction body (50) along the longitudinal direction (L), and the wiring aid (40) is arranged so that the traction body (50) is wired at an angle with respect to the longitudinal direction (L), thereby enabling at least the distal end side tubular body (20) to be curved and deformed in a desired direction .

### [Configuration 2]

A movable elongated structure which includes a plurality of tubular bodies (20 and 30) formed in a flexible, elongated shape and having an internal space (21 and 31) penetrating therein in the longitudinal direction (L), a flexible, elongated traction body, and a wiring aid (40B) arranged between two of the plurality of tubular bodies (20 and 30) arranged in series and adjacent to each other in the longitudinal direction, and maintaining a distance between the opposing ends of the tubular bodies (20 and 30), wherein the direction in which the traction body (50) is towed in the longitudinal direction (L) is the base end side (LB) and the opposite side is the distal end side (LF), wherein among the two tubular bodies (20 and 30) adjacent in the longitudinal direction in the plurality of tubular bodies (20 and 30)arranged in series, the tubular body arranged on the base end side (LB)is referred to as a base end side tubular body(30), and the tubular body arranged on the distal end side (LF)is referred to as a distal end side tubular body(20), wherein a pair of the traction bodies(50) is referred to as a traction body set (53 to 56), and four sets of the traction body sets (53 to 56)are provided, and wire lumens (22 and 32) for wiring the traction bodies (50) along the longitudinal direction (L) are provided in the tubular bodies (20 and 30), and the pair of the wire lumens (22 and 32) is referred to as wire lumen sets (23 to 26, 33 to 36), and four sets of the wire lumen sets (23 to 26, 33 to 36) are provided in which four sets of the wire lumen sets (23 to 26, 33 to 36) are arranged in four directions in the cross section of the tubular bodies (20 and 30), wherein the base end side tubular body (30) and the distal end side tubular body (20) arranged in series are arranged so that the wire lumen sets (23 to 26, 33 to 36) provided in the four directions are oriented along the longitudinal direction (L), wherein the traction body sets (23 to 26) are arranged in the distal end side tubular body (20) in one of the four directions of the cross section in which the wire lumen sets (23 to 26) are provided, wherein in the base end side tubular body, the cross section center "0" of the tubular bodies (20 and 30) in the one direction is closer to the wire lumen set arranged in the distal end side tubular body (20) than the wire lumen sets (23 to 26) arranged in the distal end side tubular body (20), and the traction bodies are arranged in the wire lumen in which the distance between the traction bodies (50) is wider, wherein in the distal end side tubular body (20), the two traction body sets (53 to 56) are arranged in the two wire lumen sets (23 and 26) that face each other in one direction across the cross-sectional center "0" of the tubular bodies (20 and 30), wherein in the base end side tubular body (30), the traction bodies (50) in the two traction body sets (53 to 56) are arranged in one of the wire lumens (32) in the two wire lumen sets (33 to 36) that face each other across the cross-sectional center "0" of the tubular body in a cross direction that crosses the one direction, and wherein in the base end side tubular body (30), a first virtual line (FL) connecting the traction bodies (50) of one (32) of the two traction body sets (53 to 56) arranged in one of the wire lumens in the two wire lumen sets (33 to 36) in the cross-sectional direction and a second virtual line (SL) connecting the traction bodies (50) of the other traction body sets (53 to 56) in the cross-sectional direction intersect in the cross-sectional direction, wherein by towing the multiple traction bodies (50) toward the base end side (LB), at least one of the base end side tubular body (30) and the distal end side tubular body (20) is curved and deformed in a desired direction relative to the other.

### [Configuration 3]

Alternatively, there may be provided a movable elongated structure (10B) in which a pair of traction bodies (50) is a traction body set (53 and 54), and two sets of traction body sets (53 and 54) are provided, a wire lumen (22 and 32) for wiring the traction bodies (50) along the longitudinal direction (L) is provided in the tubular bodies (20 and 30), the pair of wire lumens (22 and 32) is a wire lumen set (23, 24, 35, and 36), two sets of wire lumen sets (23, 24, 35, and 36) are provided in the tubular body (20 and 30), the two sets of wire lumen sets (23, 24, 35, and 36) are arranged at opposing positions in the cross section of the tubular body (20 and 30), wherein the distal end side tubular body (20) is arranged in series with the base end tubular body (30) in such a direction that the opposing directions in which the wire lumens (23 and 24) in the distal end side tubular body (20) are arranged cross the opposing directions in which the wire lumen sets (35 and 36) in the base end side tubular body (30) are arranged, wherein two sets of traction body sets (53 and 54) are arranged in the distal end side tubular body (20) to the two sets of wire lumen sets (23 and 24), wherein in the base end side tubular body (30), the traction body (50) of one (53) of the two sets of traction body sets (53 and 54) is respectively arranged to either one (35b 36b) of the wire lumen sets (35 and 36), wherein the traction body (50) of the other traction body set (54) is respectively arranged in the unrouted wire lumens (35a and 36a) of the two wire lumen sets (35 and 36), wherein in the base-end side tubular body (30), the traction bodies (50) of the two traction body sets (53 and 54) are arranged in either of the wire lumens (22 and 32) of the two wire lumen sets (35 and 36) that face each other across the cross-sectional center "0" of the tubular body (20 and 30) in a crossing direction that crosses one direction, wherein in the base-end side tubular body (30), a first imaginary line (FL) connecting the traction bodies (50) of one of the traction body sets (53) of the two traction body sets (53 and 54) arranged in any of the wire lumens (35a, 36a, 35b and 36b) in the two wire lumen sets (35 and 36) in the cross-sectional direction and a second imaginary line (SL) connecting the traction bodies (50) of the other traction body set (54) in the cross-sectional direction intersect in the cross-sectional direction, and wherein by towing the multiple traction bodies (50) toward the base end side (LB), at least one of the base end side tubular body (30) and the distal end side tubular body (20) is curved and deformed in a desired direction. relative to the other.

### [Configuration 4]

A movable elongated structure (10) includes a plurality of tubular bodies (20 and 30) each formed in a flexible, long shape and having an internal space (21 and 31) penetrating therethrough in the longitudinal direction (L), a flexible, long-shaped traction body (50), and a wiring aid (40) disposed between two longitudinally adjacent tubular bodies (20 and 30) among the plurality of tubular bodies (20 and 30) disposed in series and maintaining a distance between opposing ends of the tubular bodies (20 and 30). In the longitudinal direction (L), the direction in which the traction body (50) is towed is the base end side (LB), and the opposite side is the distal end side (LF), and the wiring aid (40) is disposed in series, wherein of the two tubular bodies (20 and 30) adjacent to each other in the longitudinal direction (L), the tubular body (20 and 30) arranged on the base end side (LB) is referred to as the base end side tubular body (30), and the tubular body (20 and 30) arranged on the distal end side (LF) is referred to as the distal end side tubular body (20), wherein four pairs of traction bodies (50) are provided as traction body sets (53 to 56), wherein a wire lumen (22 and 32) for wiring the traction body (50) along the longitudinal direction (L) is provided in the tubular bodies (20 and 30), wherein four pairs of wire lumens (22 and 32) are provided as wire lumen sets (23 to 26, 33 to 36). wherein four wire lumen sets (23 to 26, 33 to 36) are provided, wherein the wire lumen sets (23 to 26, 33 to 36) provided in the four directions (HU, HD, WR, WL) of the cross section of the tubular bodies (20 and 30) are arranged in four directions (HU, HD, WR, WL) in the base end side tubular body (30) and the distal end side tubular body (20) arranged in series are arranged in a direction along the longitudinal direction (L), wherein the wire lumen sets (23 to 26) are arranged in one of the four directions (HU, HD, WR, WL) of the cross section of the distal end side tubular body (20) in which the wire lumen sets (23 to 26) are provided, and the wire lumen sets (23 to 26) arranged in the base end side tubular body (30) are arranged in one of the four directions (HU, HD, WR, WL) of the cross section of the distal end side tubular body (20), wherein the distal end side tubular body (20) is arranged in a wire lumen (33a to 36a, 33b to 36b) that is closer to the cross-sectional center "0" of the tubular body (20, 30) in one direction (HU, HD, WR, WL) than the wire lumen (23 to 26) and in which the interval between the arranged traction bodies (53a to 56a, 53b to 56b) is wider, and wherein the distal end side tubular body (20) is curved and deformed in a desired direction relative to the base end side tubular body (30) by towing the plurality of traction bodies (53a to 56a, 53b to 56b) or the traction body (53a to 56a, 53b to 56b) and the traction body set (53 to 56) toward the base end side (LB) .

### [Configuration 5]

A movable elongated structure (10) includes a plurality of tubular bodies (20A and 30) formed in a flexible, long shape and having an internal space (21, 31) penetrating therein in the longitudinal direction (L), a long, flexible traction body (50, 50A), and a wiring aid (40A) disposed between two tubular bodies (20A, 30) adjacent in the longitudinal direction (L) among the plurality of tubular bodies (20A and 30) disposed in series and maintaining a distance between opposing ends of the tubular bodies (20A and 30), wherein in the longitudinal direction (L), the direction in which the traction body (50, 50A) is towed is the base end side (LB), and the opposite side is the distal end side (LF), wherein of the two tubular bodies (20A and 30) adjacent to each other in the longitudinal direction (L), the tubular body (20A and 30) arranged on the base end side (LB) is referred to as the base end side tubular body (30), and the tubular body (20A and 30) arranged on the distal end side (LF) is referred to as the distal end side tubular body (20A), wherein the pair of traction bodies (50, 50A) is referred to as a traction body set (53 to 56), wherein four sets of the traction body sets (53 to 56) are provided, and one of the pair of traction bodies (50) is a traction body (50) arranged across the base end side tubular body (30) and the distal end side tubular body (20A) is referred to as a long traction body (50), and the other is a short traction body (50A) arranged on the base end side tubular body (30), wherein the traction bodies (50) are arranged in the longitudinal direction (L) on the tubular bodies (20A and 30), wherein a pair of wire lumens (32) is formed as a wire lumen set (33 to 36), four wire lumen sets (33 to 36) are provided, the four wire lumen sets (33 to 36) are arranged in four directions in a cross section of the base end side tubular body (30), the four wire lumens (22A) are arranged in four directions in a cross section of the distal end side tubular body (20A), the base end side tubular body (30) and the distal end side tubular body (20A) arranged in series are arranged in such a direction that the four directions are aligned, and the long traction body (50) of the traction body set (53 to 56) is wired in the wire lumen (22A) in one of the four directions in the cross section in which the wire lumen (22A) is provided in the distal end side tubular body (20A), wherein the long traction body (50) and the short traction body (50A) of the traction body set (53 to 56) are arranged in the base end side tubular body (30) in a wire lumen (32) that is closer to the cross-sectional center "0" of the tubular body (20, 30) in one direction than the wire lumen (22A) arranged in the distal end side tubular body (20A) and faces in a direction intersecting the one direction, and the short traction body (50A) arranged in the wire lumen (32) of the base end side tubular body (30) is fixed to the base end side tubular body (30) at the distal end side (LF) of the base end side tubular body (30), and each traction body (50, 50A) is configured to be able to independently tow, and wherein by towing a plurality of traction bodies (50, 50A) toward the base end side (LB), at least one of the base end side tubular body (30) and the distal end side tubular body (20A) is curved and deformed in a desired direction relative to the other.

The first virtual line (FL) and the second virtual line (SL) may pass through the cross-sectional center "0".

In addition, the wiring aid (40B) may be provided with a regulating portion (45B) that regulates the relative positions of a traction body (50) of one traction body set (53 to 56) and a traction body (50) of the other traction body set (53 to 56) out of two traction body sets (53 to 56) that cross between adjacent tubular bodies (20 and, 30) in the longitudinal direction (L).

The traction body (50, 50A) may be a flexible wire, and the wire lumen may be a through passage provided along the longitudinal direction inside the tube wall of the tubular body.

There may be provided a movable elongated treatment instrument in which a treatment instrument such as a retractor, gripper, forceps, tweezers needle, probe, or scissors is provided at the distal end of the distal end side tubular body (20, 20A) in the above-mentioned movable elongated structure (10, 10A, 10B), and a driving mechanism for the treatment instrument may be disposed in the internal space (21). A cable for transmitting high frequency (radio wave) or microwave may be wired to the treatment instrument in order to heat, cauterize, stop bleeding, or cut biological tissue with the treatment instrument.

Of the two tubular bodies (20, 20A, and 30) arranged in series, the distal tubular body (20, 20A) may be an elastic retractor, elastic gripper, forceps or scissors having an internal space, and may be opened and closed by a wire (50).

### [Configuration 6]

A method for inserting a movable elongated structure, including: inserting the above-mentioned movable elongated structure (10, 10A, and 10B) into a pipeline; driving and controlling a traction drive unit (102, 331) that traction body (50, 50A) to bend and deform at least one of the distal end side tubular body (20, 20A) and the base end side tubular body (30) in a desired direction; and inserting the distal end side tubular body (20, 20A) into a branching pipeline.

The pipeline may be at least one of a hollow organ, a vessel, and a blood vessel.

### [Configuration 7]

A method for operating a movable elongated structure, in which the traction body set (53 to 56) of the above-mentioned movable elongated structure (10, 10A and 10B) is towed to bend and deform at least one of the distal end side tubular body (20, 20A) and the base end side tubular body (30) in a desired direction.

### [Configuration 8]

Also, in the above-mentioned movable elongated structure (10, 10A, and 10B), a method for operating a movable elongated structure is provided in which one of the plurality of traction body sets (53 to 56) is towed to bend and deform at least one of the distal end side tubular body (20, 20A) and the base end side tubular body (30) in a desired direction.

### [Configuration 9]

Also, a method for operating a movable elongated structure, in which one of the traction bodies (50) of the traction body set (53 to 56) is routed through the routing path (32) of the base end side tubular body (30) and the routing path (22A) of the distal end side tubular body (20A) and fastened to the distal end side tubular body (20A) at the distal end side (LF) of the distal end side tubular body (20A) is towed to curve and deform the distal end side tubular body (20A) in a desired direction, and the other traction body (50A) of the traction body set (53 to 56) of the traction body set (53 to 56) is routed through the routing path (32) of the base end side tubular body (30) and fastened to the base end side tubular body (30) at the distal end side (LF) of the base end side tubular body (30) to curve and deform the base end side tubular body (30) in the desired direction.

### [Configuration 10]

A movable elongated structure device (100, 317) provided with the above-mentioned movable elongated structure (10, 10A, and 10B) and a traction drive unit (102, 331) that traction the traction body (50, 50A), and the traction drive unit (102, 331) tow the traction body (50, 50A) to bend and deform at least one of the distal end side tubular body (20, 20A) and the base end side tubular body (30) in a desired direction.

### [Configuration 11]

A medical system (200) including the above-mentioned mobile elongated structural instrument (100, 317), a drive unit for driving the traction drive (102, 331), and a control unit (104, 302) connected to apply a drive signal to the drive unit.

### [Configuration 12]

A tool (317) including the above-mentioned movable elongated structural instrument (100, 317), an attachment portion for attaching the base end side tubular body (30) of the movable elongated structure (10, 10A, 10B) to the distal end of a robot arm (312), and a connection portion (361) for connecting to a drive mechanism that drives the traction drive portion (102, 331) on the robot arm (312) side.

### [Configuration 13]

A robot having the above-mentioned tool (317), a robot arm (312) with the tool (317) at its distal end, a traction drive (102, 331) and a drive unit for driving the robot arm (312), and a control unit (104, 302) connected to apply a drive signal to the drive unit.

### [Configuration 14]

A robot including: an input/output unit (310a) connected by wire and/or wirelessly to the above-mentioned movable elongated structural instrument (100, 317); an input unit that receives operation signals in real time; a calculation unit (CPU) that executes a predetermined operation program based on the operation signals; and an output unit that generates a drive signal for traction a predetermined traction body (50, 50A) using a traction drive section (102, 331) based on an output from the calculation unit (CPU) to bend and/or stretch (extend) at least the distal end side tubular body (20, 20A) in a desired direction.

### [Configuration 15]

A robot including the above-mentioned tool (317), a robot arm (312) with the tool (317) at its distal end, a traction drive (102, 331) and a drive unit for driving the robot arm (312), and a control unit (104, 302) connected to apply a drive signal to the drive unit, the control unit (104, 302) being equipped with artificial intelligence (Al).

### [Configuration 16]

A robot including the above-mentioned manipulator (100), a robot arm (312) with the manipulator (100) at its distal end, a traction drive unit (102, 331) and a drive unit for driving the robot arm (312), and a control unit (104, 302) connected to apply a drive signal to the drive unit, the control unit (104, 302) being equipped with artificial intelligence (AI).

### [Configuration 17]

A robot including: an input/output unit (310a) connected by wire and/or wirelessly to a movable elongated structural instrument (100, 317); an input unit that receives operation signals in real time; a calculation unit (CPU) that executes a predetermined operation program based on the operation signals; and an output unit that generates a drive signal that causes a predetermined traction body (50, 50A) to be towed by a traction drive unit (102, 331) based on the output from the calculation unit (CPU) to bend and/or stretch (extend) at least the distal end side tubular body (20, 20A) in a desired direction, wherein the arithmetic unit (CPU) is equipped with artificial intelligence (AI).

### [Configuration 18]

A method for operating a robot comprising the above-mentioned movable elongated structural instrument (100, 317), in which an input/output unit (310a) connected by wire and/or wirelessly receives operation signals in real time, and an arithmetic unit (CPU) executes a predetermined operation program based on the received operation signals, whereby a traction body (50, 50A) is towed by a traction drive unit (102, 331) based on the output from the arithmetic unit (CPU), thereby bending and/or stretching (extending) the distal end side tubular body (20, 20A) in a desired direction.

### [Configuration 19]

A medical robot comprising the above-mentioned robot, wherein the output unit provides a drive signal to an external drive unit that mechanically drives the movable elongated structure (10, 10A, 10B).

### [Configuration 20]

A manipulator (100) including the above-mentioned movable elongated structural instrument (100, 317) and an operating unit for operating a traction drive unit (102, 331) on the main body side.

### [Configuration 21]

A flexible endoscope is provided with the above-mentioned movable elongated structure (10, 10A, 10B) and a plurality of traction operating units (103) that traction the pair of traction bodies (50, 50A), and the traction operating units (103) tow the pair of traction bodies (50, 50A) to deform the tubular body (20A) on the distal end side (LF).

### [Configuration 22]

A steering catheter is provided with the above-mentioned movable elongated structure (10, 10A, 10B) and a plurality of traction operating units (103) that traction the pair of traction bodies (50, 50A), in which the traction operating units (103) tow the pair of traction bodies (50, 50A) to deform the tubular body (20A) on the distal end side (LF).

### [Configuration 23]

A movable elongated structure (10) provided with a plurality of tubular bodies (20 and 30) each formed in a flexible, elongated shape and having an internal space penetrating therein in the longitudinal direction (L), a flexible, elongated traction body (50), and a wiring aid (40B) disposed between two tubular bodies (20 and 30) adjacent in the longitudinal direction (L) among the plurality of tubular bodies (20 and 30) disposed in series and maintaining a distance between the opposing ends of the tubular bodies (20 and 30), and the direction in which the traction body (50) is towed in the longitudinal direction (L) is the base end side (LB), and the opposite side is the distal end side (LF), wherein among the two tubular bodies (20 and 30) adjacent in the longitudinal direction (L) in the multiple tubular bodies (20 and 30) arranged in series, the tubular body (20 and 30) arranged on the base end side (LB) is the base end side tubular body (30), and the tubular body (20 and 30) arranged on the distal end side (LF) is the distal end side tubular body (20), and a wire lumen (22 and 32) for wiring a traction body (50) along the longitudinal direction (L) is provided in the tubular bodies (20 and 30), and the pair of traction bodies (50) is traction body sets (53 to 56), a pair of wire lumens (22 and 32) is arranged as a wire lumen set (33 to 36), wherein in the base end side tubular body (30), the wire lumens (32) are arranged in a wire lumen (32) that is closer to the cross-sectional center "0" of the tubular body (20, 30) in one direction and has a wider interval between the wire lumens (50) than the wire lumen set (33 to 36) arranged in the distal end side tubular body (20), and the two sets of wire lumens (32) arranged in either of the two wire lumen sets (33 to 36) in the base end side tubular body (30) are arranged in a wire lumen (32) that is closer to the cross-sectional center "0" of the tubular body (20 and 30) in one direction and has a wider interval between the wire lumens (50). wherein a first virtual line (FL) connecting the traction bodies (50) of one of the traction body sets (53 to 56) in the cross-sectional direction and a second virtual line (SL) connecting the traction bodies (50) of the other traction body set (53 to 56) in the cross-sectional direction intersect in the cross-sectional direction, and wherein by towing the multiple traction bodies (50) toward the base end side (LB), at least one of the base end side tubular body (30) and the distal end side tubular body (20) is curved and deformed in a desired direction relative to the other .

In the following examples, a medical device will be described as an example of the treatment instrument of the present invention, but the present invention provides a treatment instrument that is not limited to a medical device.

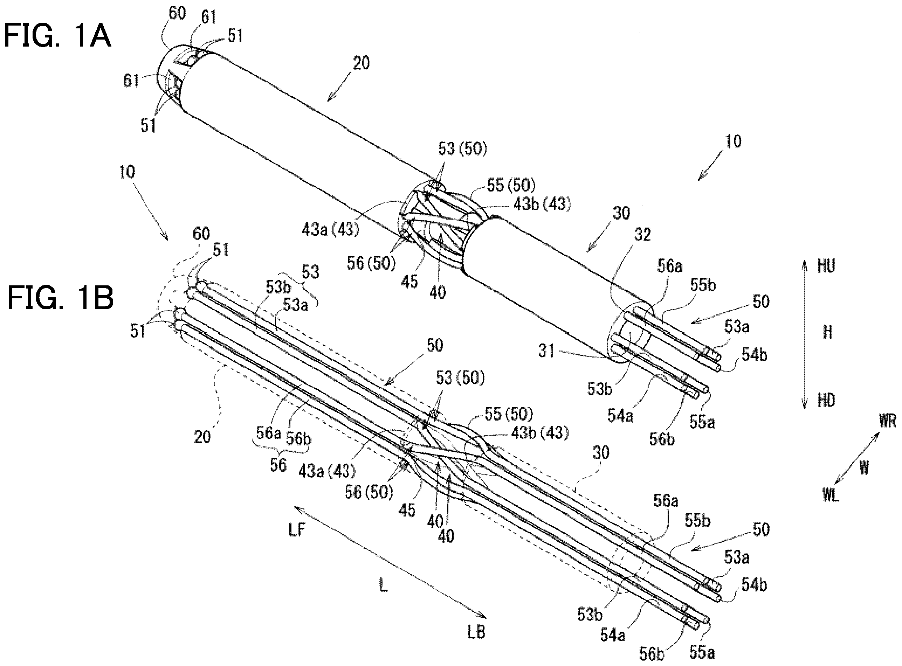

FIGS. 1 to 6 show structural diagrams of a movable elongated structure 10 as one embodiment of the present invention.

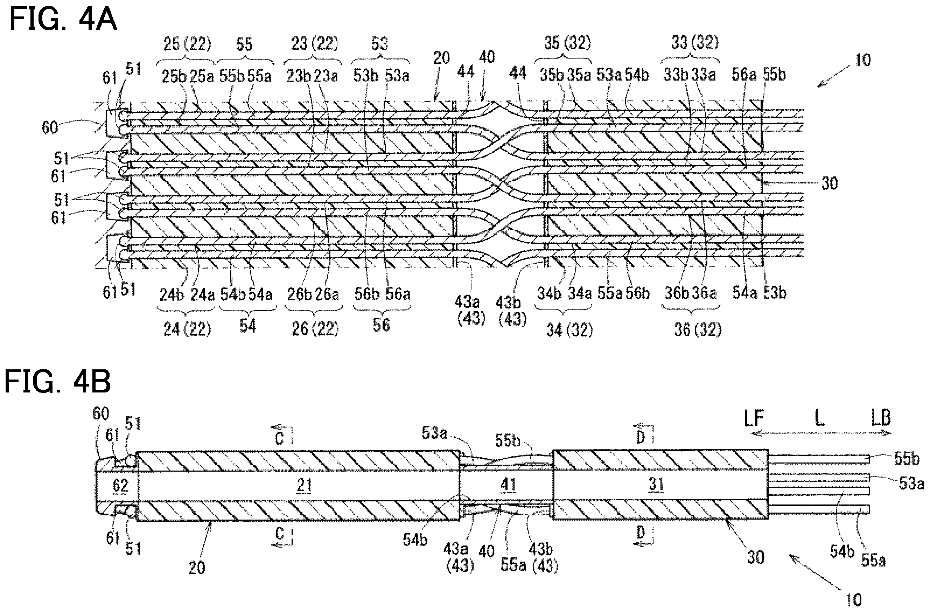

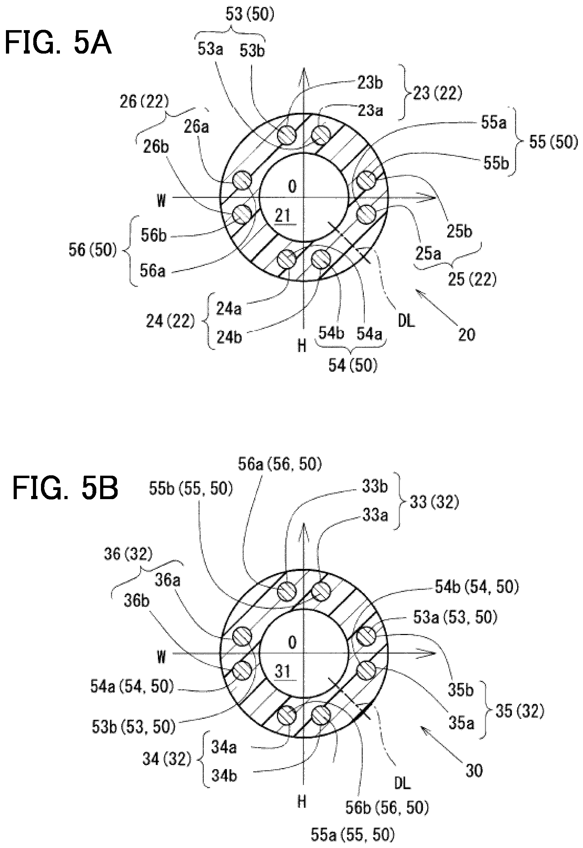

FIG. 4A is a development view of the flexible tubes 20 and 30 having an annular cross section, developed along the imaginary dividing line DL shown in FIG. 5A and FIG. 5B and illustrating the portion passing through the center of the wire lumens 22 and 32.

The movable elongated structure 10 includes a distal end side flexible tube 20 and a base end side flexible tube 30 arranged along the longitudinal direction L, a spacer (viz. wiring aid) 40 arranged between the distal end side flexible tube 20 and the base end side flexible tube 30 (hereinafter collectively referred to as the flexible tubes 20 and 30), and a traction wire 50 inserted into the tube walls of the flexible tubes 20, 30. The exterior of the movable elongated structure 10 along the longitudinal direction L is covered with an exterior cover (not shown). Alternatively, only the exterior of the spacer 40 may be covered with an exterior cover (not shown).

The distal end side flexible tube 20 is a tubular flexible tube that is long in the longitudinal direction L, and includes a main lumen 21 (FIGS. 4B and 5A) therein. The main lumen 21 is a space that is circular in cross section along the longitudinal direction L.

As shown in FIGS. 4B and 5A, the distal end side flexible tube 20 is provided with distal end side wire lumens 22 (23a to 26a, 23b to 26b) inside the tube wall between the main lumen 21 and the outer circumferential surface.

The distal end side wire lumen 22 is a space with a circular cross section extending in the longitudinal direction L inside the tube wall, and is formed with a diameter that allows the traction wire 50 described later to be inserted therethrough.

The distal end side wire lumens 22 are provided in pairs on each of the four directions (up, down, left, and right) of the tube wall having a ring-shaped cross section.

Specifically, as shown in FIGS. 4B and 5A, the tube wall has a ring-shaped cross section and is provided with distal end side wire lumens 23a and 23b in the upward direction HU, distal end side wire lumens 24a and 24b in the downward direction HD, distal end side wire lumens 25a and 25b on the right side WR, and distal end side wire lumens 26a and 26b on the left side WL, which are spaced a predetermined distance apart in the circumferential direction of the circular cross section.

In addition, when looking at the movable elongated structure 10 from the base end side LB to the distal end side LF, the clockwise side of the distal end wire lumen 22, which is composed of a pair of two through holes, is designated as 23a, 24a, 25a, and 26a, and the counterclockwise side is designated as 23b, 24b, 25b, and 26b.

In addition, the distal end side wire lumens 22 provided in two on each of the four directions (up, down, left, and right) may be integrally formed into an elliptical shape.

Of the distal end side wire lumens 22 configured in this manner, the pair of distal end side wire lumens 23a and 23b that are provided in the upward direction HU in the tube wall that has a ring-shaped cross section are collectively referred to as the distal end side upper lumen set 23, and similarly, the distal end side wire lumens 24a and 24b in the downward direction HD are referred to as the distal end side lower lumen set 24, the distal end side wire lumens 25a and 25b on the right side WR are referred to as the distal end side right lumen set 25, and the distal end side wire lumens 26a and 26b on the left side WL are referred to as the distal end side left lumen set 26.

The distal end side flexible tube 20 configured as described above may be configured from a flexible tube made of, for example, polyamide elastomer, expanded polytetrafluoroethylene, polyurethane, or polytetrafluoroethylene.

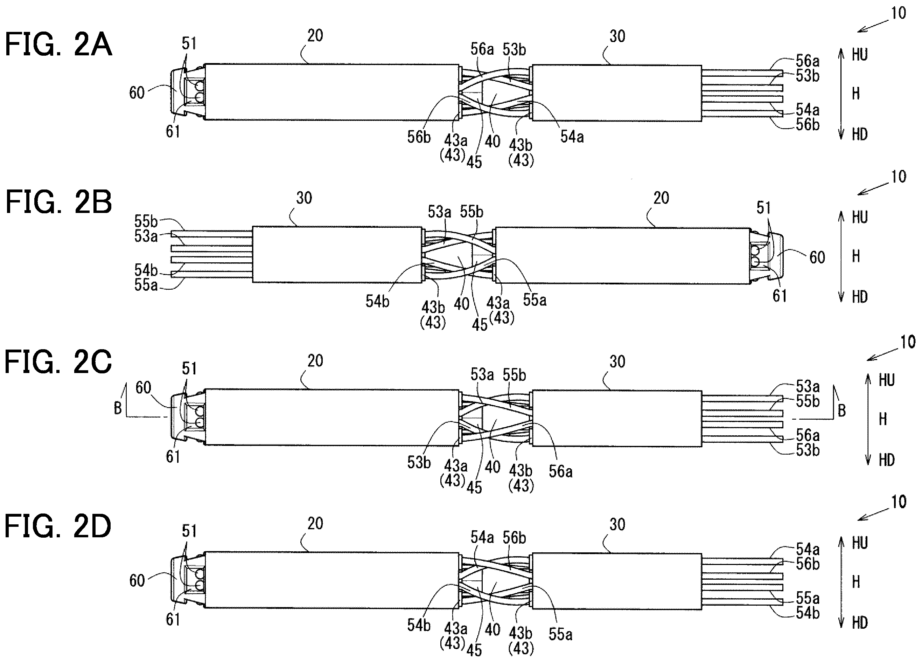

As shown in FIGS. 2 and 4, the distal end side flexible tube 20 is provided with a distal cap 60 at the end of the distal side LF.

The distal end cap 60 has a fixing recess 61 for forming an expanded diameter portion 51 of the traction wire 50 described later. The fixing recess 61 is provided at four locations corresponding to the distal end side wire lumens 22 provided in four directions (up, down, left, and right directions) in the tube wall, and has two insertion holes for inserting the traction wire 50 therethrough.

As shown in FIG. 4B, the distal end cap 60 has a through hole 62 (see FIG. 4B) that penetrates in the longitudinal direction L by an instrument or the like that is inserted into the main lumen formed by the main lumen 21, the internal space 41 described later, and the base-end main lumen 31 communicating with each other in an instrument that uses the movable elongated structure 10, but this does not necessarily have to be the case.

The base end side flexible tube 30, like the distal end side flexible tube 20, is a tubular flexible tube that is long in the longitudinal direction L, and includes a base end side main lumen 31 therein. The base end side main lumen 31 is a space that is circular in cross section along the longitudinal direction L.

As shown in FIG. 5B, the base end side flexible tube 30 is provided with a base end wire 32 (33 to 36) inside the tube wall between the base end main lumen 31 and the outer circumferential surface.

The base end side wire lumen 32, like the distal end wire lumen 22, is a space with a circular cross section extending in the longitudinal direction L inside the tube wall and is formed with a diameter that allows the traction wire 50 described later to be inserted therethrough.

The base end side wire lumen 32, like the distal end wire lumen 22, is provided in four directions (up, down, left and right) in the tube wall having a ring-shaped cross section.

Specifically, as shown in FIG. 5B, the tube wall has a ring-shaped cross section and is provided with base end side wire lumens 33a and 33b in the upward direction HU, base end side wire lumens 34a and 34b in the downward direction HD, base end side wire lumens 35a and 35b on the right side WR, and base end side wire lumens 36a and 36b on the left side WL, which are spaced a predetermined distance apart in the circumferential direction of the circular cross section.

In addition, when looking at the movable elongated structure 10 from the base end side LB to the distal end side LF, the clockwise side of the base end side wire lumen 32, which is composed of a pair of two through holes, is designated as 33a, 34a, 35a, and 36a, and the counterclockwise side is designated as 33b, 34b, 35b, and 36b.

In addition, the base end side wire lumens 32 provided in two in each of the four directions (up, down, left, and right directions) may be integrally formed into an elliptical shape.

Of the base end side wire lumens 32 configured in this manner, the pair of base end side wire lumens 33a and 33b are provided in the upward direction HU in the tube wall having a ring-shaped cross section and are collectively referred to as the base end upper lumen set 33. Similarly, the base end side wire lumens 34a and 34b in the downward direction HD are referred to as a base end side lower lumen set 34, the base end side wire lumens 35a and 35b on the right side WR are referred to as a base end right lumen set 35, and the base end side wire lumens 36a and 36b on the left side WL are referred to as a base end left lumen set 36.

The base end side flexible tube 30 configured as described above may be made of a flexible tube such as polyamide elastomer, expanded polytetrafluoroethylene, polyurethane, or polytetrafluoroethylene, similar to the distal end side flexible tube 20. The distal end side flexible tube 20 and the base end side flexible tube 30 may be made of the same material or different materials.

In the flexible tubes 20 and 30 thus constructed, the arrangement of the wire lumens 22 and 32 will be described in detail with reference to FIGS. 5A and 5B.

Specifically, as shown in FIGS. 5A and 5B in a cross section of the flexible tubes 20 and 30 viewed from the base end side LB to the distal end side LF, the arrangement of the wire lumens 22 and 32 in virtual coordinates is described, where W coordinate indicates the width direction W passing through the center "0" of the cross section, and H coordinate indicates the height direction H.

In the virtual coordinate system shown in FIGS. 5A and 5B, the right side WR in the W coordinate system is the +side, the left side WL is the - side, and the upward direction HU in the H coordinate system is the +side, and the downward direction HD is the -side.

The lumen sets 23 and 33 arranged in the upward direction HU of the flexible tubes 20 and 30 are arranged on the +side of the H coordinate, the wire lumens 23a and 33a are arranged on the +side of the W coordinate, and the wire lumens 23b and 33b are arranged on the -side of the W coordinate.

The lumen sets 24 and 34 arranged in the downward direction HD of the flexible tubes 20 and 30 are arranged on the -side of the H coordinate, the wire lumens 24a and 34a are arranged on the -side of the W coordinate, and the wire lumens 24b and 34b are arranged on the +side of the W coordinate.

The lumen set 25 and 35 located on the right side WR of the flexible tubes 20 and 30 is located on the +side of the W coordinate, the wire lumens 25a and 35a are located on the -side of the H coordinate, and the wire lumens 25b and 35b are located on the +side of the H coordinate.

The lumen set 26 and 36 arranged on the left side WL of the flexible tubes 20 and 30 is arranged on the -side of the W coordinate, the wire lumens 26a and 36a are arranged on the +side of the H coordinate, and the wire lumens 26b and 36b are arranged on the -side of the H coordinate.

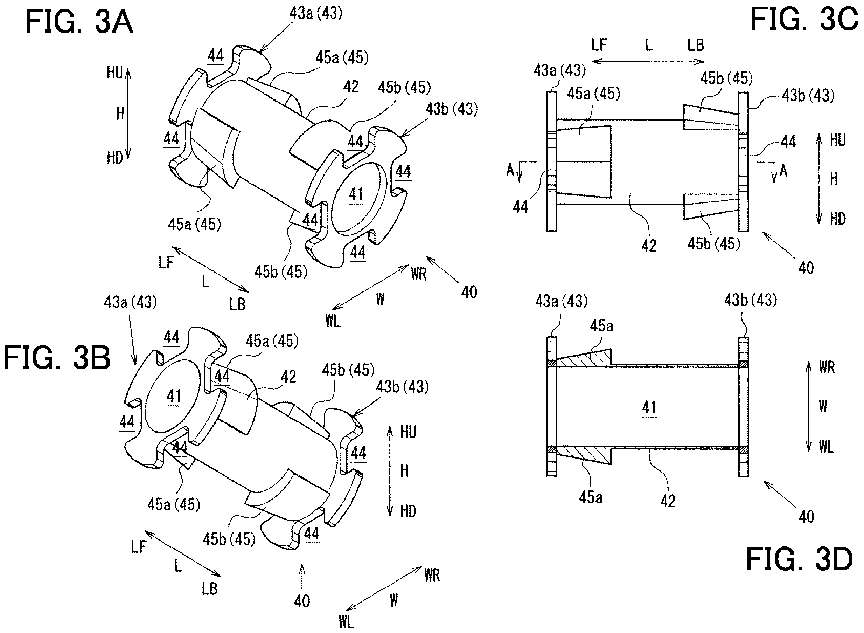

As shown in FIG. 3, the spacer 40 includes a cylinder 42 having an internal space 41 with a circular cross section penetrating therethrough in the longitudinal direction L, and protruding edge portions (viz. flange) 43 (43a and 43b) provided at both ends of the cylinder 42 in the longitudinal direction L.

The internal space 41 is formed with a space having the same diameter as the above-mentioned main lumens 21 and 31. The cylinder 42 is formed with a diameter arranged on the radially inner side of the traction wire 50 inserted into the distal end side wire lumen 22 provided in the distal end side flexible tube 20 and the base end side wire lumen 32 provided in the base end side flexible tube 30.

The protruding edge portions 43 (43a and 43b) are formed with an outer diameter that protrudes radially outward from the outer diameter of the cylinder 42, and are provided with an arrangement recess 44 for arranging the traction wire 50 at locations corresponding to the distal end side wire lumen 22 and the base end side wire lumen 32 (hereinafter referred to as wire lumens 22, 32). The protruding edge portions 43 are formed with an outer diameter equivalent to the outer diameters of the distal end side flexible tube 20 and the base end side flexible tube 30.

The placement recess 44 is a recess for collectively placing two traction wires 50 which are inserted into the two wire lumens 22 and 32 provided, and is formed with a width and depth corresponding to the two wire lumens 22 and 32. Of the protruding edge portions 43 provided on both sides of the longitudinal direction L of the cylinder 42, the distal end side LF is a distal end side protruding edge portion 43a, and the base end side LB is a base end side protruding edge portion 43b.

Furthermore, a guide protrusion 45 is provided at the end of the cylinder 42 in the longitudinal direction L to guide the traction wire 50 radially outward. The guide protrusion 45 is provided at the end of the longitudinal direction L at a position corresponding to the placement recess 44 of the protruding edge portion 43, with a corresponding width, and is formed so that its cross-sectional shape is trapezoidal, with the protruding height gradually increasing from the end of the longitudinal direction L toward the center of the longitudinal direction L of the cylinder 42 (see FIG. 3D).

The guide protrusions 45 are provided at both ends in the longitudinal direction L at radially opposing positions among the arrangement recesses 44 provided in four directions (up, down, left, and right), and are provided so that the opposing directions are perpendicular to the distal end side guide protrusion 45a on the distal end side LF and the base end side guide protrusion 45b on the base end side LB.

Specifically, the distal end side guide protrusions 45a on the distal end side LF are provided in the upward direction HU and the downward direction HD so as to face each other in the height direction H, and the base end side guide protrusions 45b on the base end side LB are provided on the right side WR and the left side WL so as to face each other in the width direction W.

The spacer 40 configured as described above may be made of, for example, a metal material such as stainless steel, an elastic body such as polytetrafluoroethylene, an elastic body such as a spring, or a resin material.

In addition, the spacer 40 may be configured such that the cylinder 42 and the protruding edge portion 43 are integral with each other, or may be configured as separate bodies and then assembled. When the cylinder 42 and the protruding edge portion 43 are configured as separate bodies, the cylinder 42 and the protruding edge portion 43 may be configured from the same material or from different materials.

In addition, the spacer 40 may be configured to protrude in the longitudinal direction L from the protruding edge portion 43 and engage with the main lumens 21 and 31 of the distal end side flexible tube 20 and the base end side flexible tube 30 so that the spacer 40 does not move radially relative to the distal end side flexible tube 20 and the base end side flexible tube 30.

The traction wire 50 is a flexible wire having an enlarged diameter portion 51 at its distal end and a length slightly longer than the length of the movable elongated structure 10 in the longitudinal direction L.

There are eight traction wires 50 each having an enlarged diameter portion 51 at its distal end.

More specifically, eight traction wires 50 are provided corresponding to the wire lumens 22 and 32 provided in the four directions (up, down, left, and right directions).

Specifically, the traction wires 50 include eight traction wires: traction wires 53a and 53b arranged in the distal end side upper lumen set 23 in the upward direction HU of the distal end side flexible tube 20 , traction wires 54a and 54b arranged in the distal end side lower lumen set 24 in the downward direction HD, traction wires 55a and 55b arranged in the distal end side right lumen set 25 disposed on the right side WR, and traction wires 56a and 56b arranged in the distal end side left lumen set 26 on the left side WL.

In addition, the traction wires 53a and 53b are arranged corresponding to the distal end side wire lumens 23a and 23b of the distal end upper lumen set 23 in the upward direction HU.

Similarly, traction wires 54a and 54b are arranged corresponding to the distal end side wire lumens 24a and 24b of the distal end side lower lumen set 24 in the downward direction HD, traction wires 55a and 55b are arranged corresponding to the distal end side wire lumens 25a and 25b of the distal end side right lumen set 25 on the right side WR, and traction wires 56a and 56b are arranged corresponding to the distal end side wire lumens 26a and 26b of the distal end side left lumen set 26 on the left side WL.

Of the traction wires 50 configured in this manner, the pair of traction wires 53a and 53b are collectively referred to as a first wire set 53. Similarly, the pair of traction wires 54a and 54b are collectively referred to as a second wire set 54, the pair of traction wires 55a and 55b are collectively referred to as a third wire set 55, and the pair of traction wires 56a and 56b are collectively referred to as a fourth wire set 56.

The traction wire 50 may be made of a metal material such as stainless steel, nylon, fluorocarbon, or the like.

In addition, the traction wires 50 do not have to be eight, and may be formed by bending four long traction wires 50.

The assembly of the movable elongated structure 10 having the above-described components will be described below.

First, from the distal end LF toward the base end LB, the distal end side flexible tube 20, the spacer 40, and the base end side flexible tube 30 are arranged in series in this order along the longitudinal direction L. At this time, the distal end side flexible tube 20 and the base end side flexible tube 30 are arranged in orientations corresponding to the wire lumens 22 and 32 provided in the four directions (up, down, left, and right).

As shown in FIGS. 1 and 4, the distal end side wire lumens 22 (23 to 26), the arrangement recesses 44 in the four directions (up, down, left, and right), and the base end side wire lumens 32 (33 to 36) are arranged so as to be connected in the longitudinal direction L.

Then, the eight traction wires 50 are inserted through the distal end side wire lumen 22, the placement recess 44, and the base end side wire lumen 32.

Specifically, in the distal end side flexible tube 20, the first wire set 53 is arranged in the distal upper lumen set 23 in the upward direction HU among the four directions of the cross section in which the distal end side wire lumen 22 is provided. Then, in the base end side flexible tube 30, the first wire set 53 is arranged in the base end side wire lumens 35b and 36a which are closer to the cross-sectional center "0" in the height direction H than the distal upper lumen set 23 arranged in the distal end side flexible tube 20, and the interval between the arranged traction wires 53a and 53b in the width direction W is wider. Note that, of the lumen sets 35 and 36 facing each other in the width direction W, the base end side wire lumens 35b and 36a are arranged on the +side of the H coordinate similarly to the distal upper lumen set 23 in the distal end side flexible tube 20.

In addition, in the spacer 40 arranged between the distal end side flexible tube 20 and the base end side flexible tube 30, the traction wires 53a and 53b are arranged in appropriate arrangement recesses 44 in the protruding edge portion 43 so that the first wire set 53 may be arranged as described above.

In addition, the expanded diameter portions 51 of the traction wires 53a and 53b are disposed in the fixed recesses 61 in the upward direction HU of the distal end cap 60 in the distal end side flexible tube 20. This allows the towing force for towing the first wire group 53 to act on at least the distal end side flexible tube 20.

In the distal end side flexible tube 20, the second wire set 54 is arranged in the distal lower lumen set 24 in the downward direction HD among the four directions of the cross section in which the distal end side wire lumen 22 is provided. Then, in the base end side flexible tube 30, the second wire set 54 is arranged in the base end side wire lumens 35a and 36b that are closer to the cross-sectional center "0" in the height direction H than the distal lower lumen set 24 arranged in the distal end side flexible tube 20, and the interval between the arranged traction wires 54a and 54b in the width direction W is wider. Note that, of the lumen sets 35 and 36 facing each other in the width direction W, the base end side wire lumens 35a and 36b are arranged on the negative side of the H coordinate, similar to the distal lower lumen set 24 in the distal end side flexible tube 20.

Similarly, in the spacer 40 arranged between the distal end side flexible tube 20 and the base end side flexible tube 30, the traction wires 54a and 54b are arranged in appropriate arrangement recesses 44 in the protruding edge portion 43 so that the second wire set 54 may be arranged as described above. In addition, the expanded diameter portions 51 of the traction wires 54a and 54b are disposed in the fixed recesses 61 in the downward direction HD of the distal end cap 60 in the distal end side flexible tube 20. This allows the towing force for towing the second wire set 54 to act on at least the distal end side flexible tube 20.

In the distal end side flexible tube 20, the third wire set 55 is arranged in the distal right lumen set 25 on the right side WR among the four directions of the cross section in which the distal end side wire lumen 22 is provided. Then, in the base end side flexible tube 30, the third wire set 55 is arranged in the base end side wire lumens 33a and 34b that are closer to the cross-sectional center "0" in the width direction W than the distal right lumen set 25 arranged in the distal end side flexible tube 20, and the interval between the arranged traction wires 55a and 55b in the height direction H is wider. In addition, the base end side wire lumens 33a and 34b of the lumen sets 23 and 24 facing each other in the height direction H are arranged on the + side of the W coordinate, similar to the distal right lumen set 25 in the distal end side flexible tube 20.

Similarly, in the spacer 40 arranged between the distal end side flexible tube 20 and the base end side flexible tube 30, the traction wires 55a and 55b are arranged in appropriate arrangement recesses 44 in the protruding edge portion 43 so that the third wire set 55 may be arranged as described above.

In addition, the expanded diameter portion 51 of the traction wires 55a and 55b is disposed in the fixed recess 61 on the right side WR of the distal end cap 60 of the distal end side flexible tube 20. This allows the towing force for towing the third wire group 55 to be applied to at least the distal end side flexible tube 20.

In the distal end side flexible tube 20, the fourth wire set 56 is arranged in the distal left lumen set 26 on the left side WL among the four directions of the cross section in which the distal end side wire lumen 22 is provided. Then, in the base end side flexible tube 30, the fourth wire set 56 is arranged in the base end side wire lumens 33b and 34a that are closer to the cross-sectional center "0" in the width direction W than the distal left lumen set 26 arranged in the distal end side flexible tube 20, and the interval between the arranged traction wires 56a and 56b in the height direction H is wider. Note that, of the lumen sets 23 and 24 facing each other in the height direction H, the base end side wire lumens 33a and 34b are arranged on the negative side in the W coordinate, similar to the distal left lumen set 26 in the distal end side flexible tube 20.

Similarly, in the spacer 40 disposed between the distal end side flexible tube 20 and the base end side flexible tube 30, the traction wires 56a and 56b are disposed in appropriate recesses 44 in the protruding edge portion 43 so that the fourth wire set 56 may be arranged as described above.

In addition, the expanded diameter portions 51 of the traction wires 56a, 56b are disposed in the fixed recesses 61 on the left side WL of the distal end cap 60 in the distal end side flexible tube 20. This allows the towing force that tows the fourth wire group 56 to act on at least the distal end side flexible tube 20.

In the above description, the wire sets (53 to 56) are arranged in this order, but the order in which the wires are arranged is not limited, and the wires may be arranged in any order.

Furthermore, after the traction wire 50 is arranged in the distal end side wire lumen 22 of the distal end side flexible tube 20 and the base end side wire lumen 32 of the base end side flexible tube 30 arranged along the longitudinal direction L, the traction wire 50 between the distal end side flexible tube 20 and the base end side flexible tube 30 is loosened, a spacer 40 is arranged between the distal end side flexible tube 20 and the base end side flexible tube 30, and the loosened traction wire 50 is arranged in the arrangement recess 44.

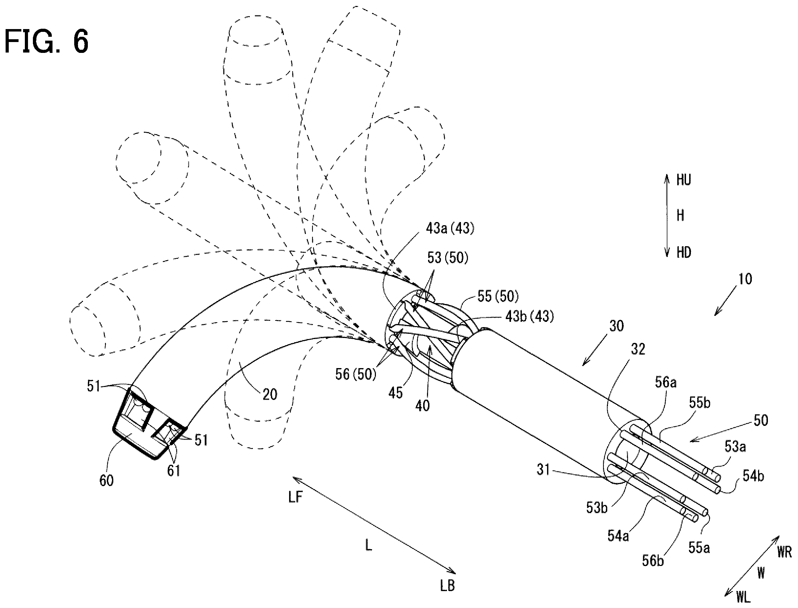

The movable elongated structure 10 configured in this manner may be smoothly curved and deformed in the longitudinal direction L by towing the traction wire 50 toward the base end side LB, as shown in FIG. 6.

Specifically, the distal end side flexible tube 20 may be bent in the upward direction HU by towing the first wire group 53 arranged in the distal end side upper lumen group 23 in the upward direction HU toward the base end side LB, and the distal end side flexible tube 20 may be bent in the downward direction HD by towing the second wire group 54 arranged in the distal end side lower lumen group 24 in the downward direction HD toward the base end side LB.

Furthermore, by towing the third wire group 55 arranged in the distal end right lumen group 25 of the right side WR in the distal end side flexible tube 20 toward the base end LB, the distal end side flexible tube 20 may be bent to the right side WR, and by towing the fourth wire group 56 arranged in the distal end left lumen group 26 of the left side WL toward the base end LB, the distal end side flexible tube 20 may be bent to the left side WL.

In this way, by towing each of the wire groups (53 to 56) arranged in the distal end lumen groups (23 to 26) of the distal end side flexible tube 20, the distal end side flexible tube 20 may be bent and deformed in the four directions (up, down, left, and right) of the arrangement direction of the distal end lumen group (23 to 26) to which the traction wire group (53 to 56) is arranged.

In response to this, by towing some of the traction wires (53a to 56a, 53b to 56b) constituting the wire set (53 to 56), the distal end side flexible tube 20 may be bent and deformed in a desired direction.

For example, by towing a traction wire 53a arranged in the distal end side wire lumen 23a of the distal end upper lumen set 23 in the upward direction HU and a traction wire 55b arranged in the distal end side wire lumen 25b of the distal end right lumen set 25 in the right side WR, the distal end side flexible tube 20 and the base end side flexible tube 30 may be bent and deformed diagonally upward and to the right, which is the upward direction HU and the right side WR.

In response to this, by towing the first wire set 53 arranged in the distal end side upper lumen set 23 in the upward direction HU and the traction wire 55b arranged in the distal end side wire lumen 25b of the distal end side right lumen set 25 on the right side WR, the distal end side flexible tube 20 may be bent and deformed in the diagonally upper right direction which is the upward direction HU and the right side WR, and in the upper right direction which is the upward direction HU.

Conversely, by towing the traction wire 53a arranged in the distal end side wire lumen 23a of the distal end upper lumen set 23 in the upward direction HU and the third wire set 55 arranged in the distal end right lumen set 25 on the right side WR, the distal end side flexible tube 20 may be bent and deformed in the diagonally upward-right direction which is the upward direction HU and the right side WR, and further in the upward-right-right direction which is the right side WR.

Similarly, by towing the respective traction wires 50 arranged in the distal end side wire lumen 23b of the distal end upper lumen set 23 in the upward direction HU and the distal end side wire lumen 26a of the distal end left lumen set 26 on the left side WL, the distal end side wire lumen 26a of the distal end upper lumen set 23 in the upward direction HU and the distal end left lumen set 26 on the left side WL, or the distal end side wire lumen 23b of the distal end upper lumen set 23 in the upward direction HU and the distal end left lumen set 26 on the left side WL, the distal end side flexible tube 20 may be bent and deformed diagonally upward and to the left.

In addition, by towing each of the traction wires 50 arranged in the distal end side wire lumen 24b of the distal end lower lumen set 24 in the downward direction HD and the distal end side wire lumen 25a of the distal end right lumen set 25 of the right side WR, the distal end side wire lumen 25a of the distal end lower lumen set 24 in the downward direction HD and the distal end right lumen set 25 of the right side WR, or the distal end side wire lumen 24b of the distal end lower lumen set 24 in the downward direction HD and the distal end right lumen set 25 of the right side WR, the distal end side flexible tube 20 may be bent and deformed diagonally downward and to the right.

Furthermore, by towing each of the traction wires 50 arranged in the distal end side wire lumen 24b of the distal end lower lumen set 24 in the downward direction HD and the distal end side wire lumen 26a of the distal end left lumen set 26 on the left side WL, the distal end side wire lumen 26a of the distal end lower lumen set 24 in the downward direction HD and the distal end left lumen set 26 on the left side WL, or the distal end side wire lumen 24b of the distal end lower lumen set 24 in the downward direction HD and the distal end left lumen set 26 on the left side WL, the distal end side flexible tube 20 may be bent and deformed diagonally downward and to the left.

In this manner, in the movable elongated structure (10), the expanded diameter portions 51 of the eight traction wires 50 are disposed in the fixed recesses 61 of the distal end cap 60 on the distal end side of the distal end side flexible tube (20), and in the distal end side flexible tube (20), the lumen sets (23 to 26) are arranged in one of the four directions (upward direction HU, downward direction HD, right side WR, left side WL) of the cross section in which the lumen sets (23 to 26) are provided, and in the base end side flexible tube (30), the lumen sets (23 to 26) are arranged in one direction (upward direction H) more than the lumen sets (23 to 26) arranged in the distal end side flexible tube (20). Since the traction bodies (53a to 56a, 53b to 56b) are arranged in the base end side wire lumens (33a to 36a, 33b to 36b) in which the distance to the cross-sectional center "0" in the directions U, downward HD, right side WR, and left side WL is short and the distance between the arranged traction bodies (53a to 56a, 53b to 56b) is wide, the distal end side flexible tube 20 may be bent and deformed in a desired direction relative to the base end side flexible tube 30 by towing the traction bodies (53a to 56a, 53b to 56b) or the traction body (53a to 56a, 53b to 56b) and the traction body set (53 to 56) toward the base end side LB.

In addition, when towing the distal end traction wires 50, the towing forces may be the same, or each traction wire 50 may be towed with a different towing force.

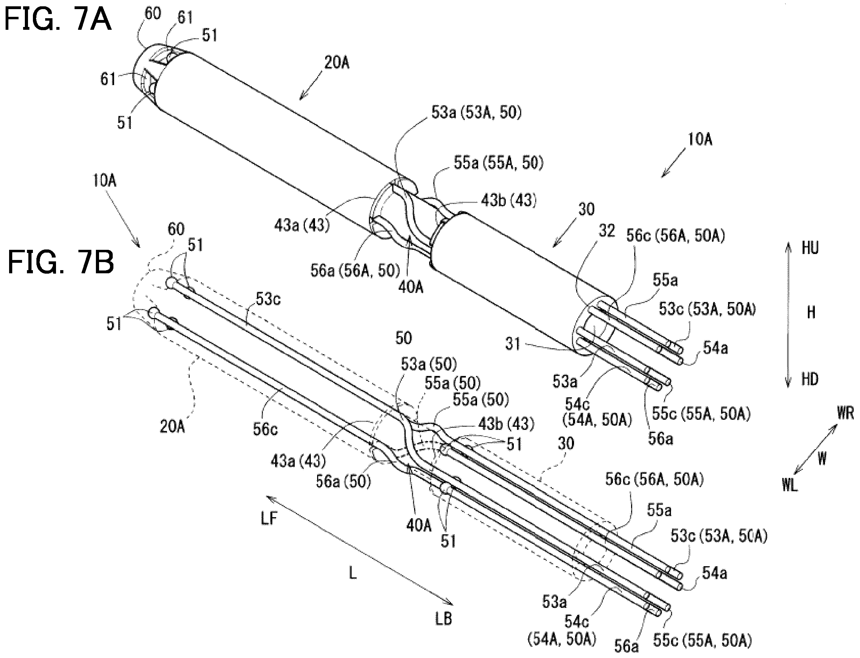

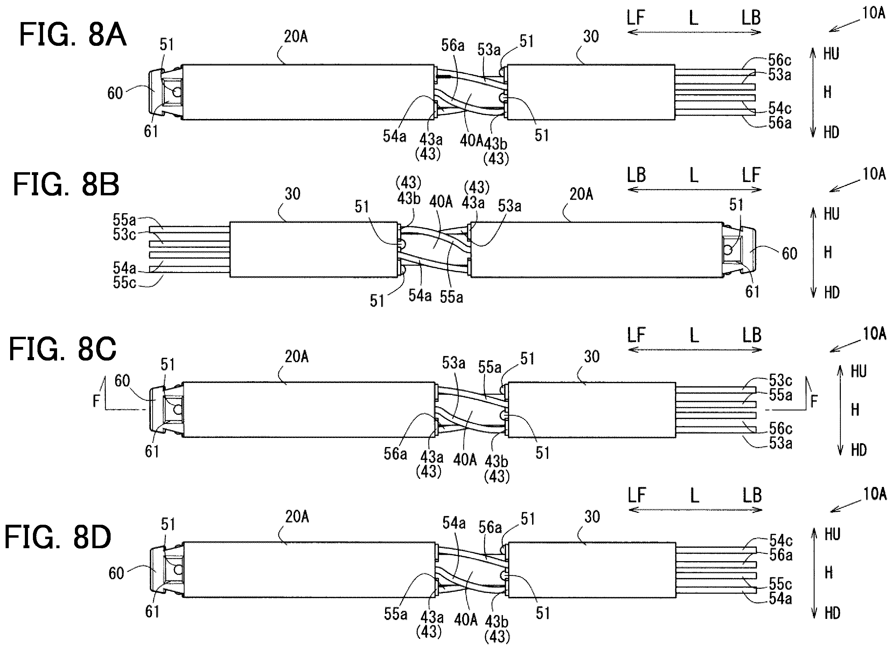

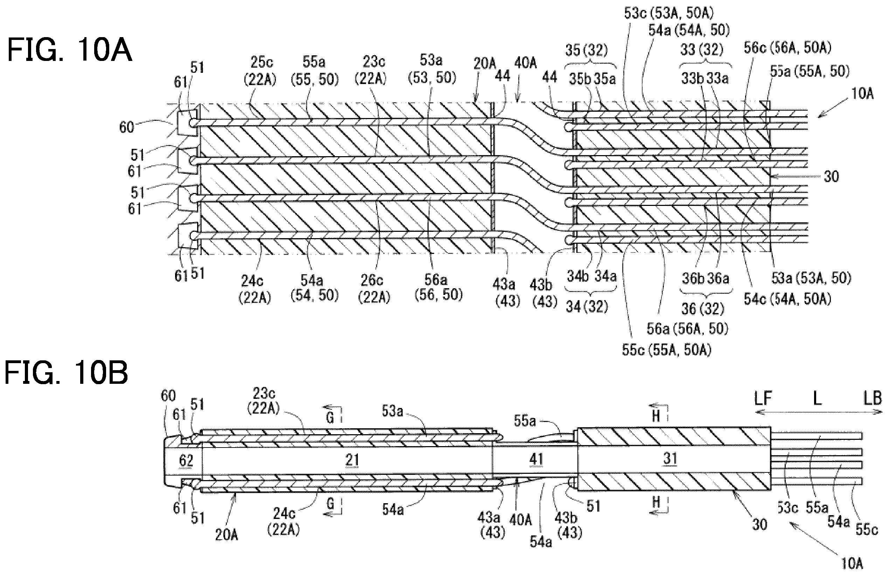

Next, a movable elongated structure 10A according to a different embodiment will be described with reference to FIGS. 7 to 12.

The same components as those in the above-described movable elongated structure 10 are denoted by the same reference numerals, and description of those components will be omitted. In the following, the components of the movable elongated structure 10A that are different from those in the above-described movable elongated structure 10 will be described.

Unlike the movable elongated structure 10 having the above-mentioned distal end side flexible tube 20, base end side flexible tube 30, spacer 40 and eight traction wires 50, the movable elongated structure 10A has, in addition to the distal end side flexible tube 20A, base end side flexible tube 30, spacer 40A and four traction wires 50, four short traction wires 50A that are shorter than the traction wires 50.

The distal end side flexible tube 20A has a main lumen 21 at the cross-sectional center "0", and unlike the distal end side flexible tube 20 which has lumen sets (23 to 26) each consisting of a pair of distal end side wire lumens 22 in four directions (up, down, left, right) inside the tube wall, the distal end side flexible tube 20A has distal end side wire lumens 22A (23c to 26c) in four directions (up, down, left, and right).

In addition, the base end side flexible tube 30 in the movable elongated structure 10A has a base end lumen set (33 to 36) consisting of a pair of base end side wire lumens 32 in four directions (up, down, left, and right), similar to the base end side flexible tube 30 described above .

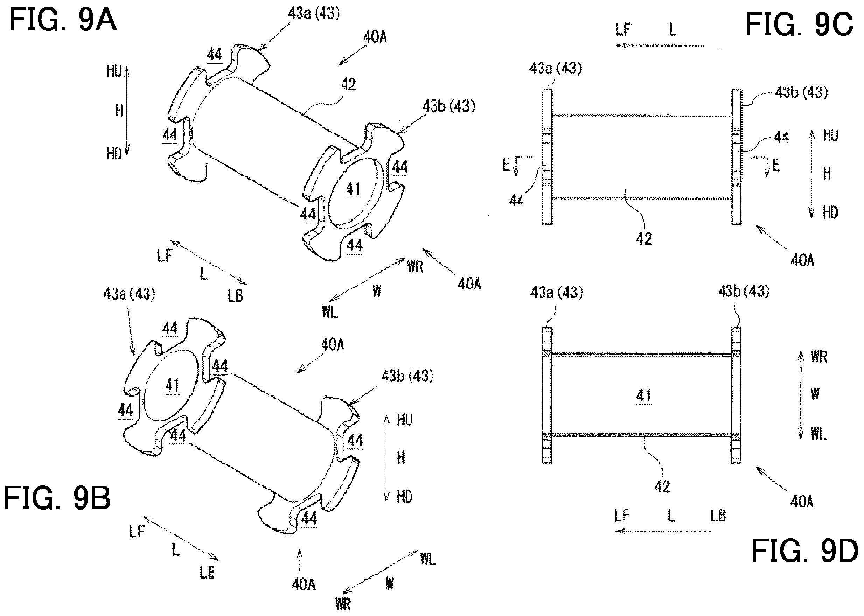

As shown in FIGS. 9A to 9D, spacer 40A is different from the spacer 40 in the movable elongated structure 10 in that it does not include the guiding protrusion 45, but the rest of the configuration is the same.

In addition, as described above, in contrast to the movable elongated structure 10 having eight traction wires 50, the movable elongated structure 10A has eight traction wires, including four traction wires 50 (53a, 54a, 55a and 56a) and four short traction wires 50A (53c, 54c, 55c and 56c).

As described above, the traction wire 50 (53a, 54a, 55a and 56a) is formed longer than the movable elongated structure 10 in order to be arranged across the distal end side wire lumen 22A of the distal end side flexible tube 20 and the base end side wire lumen 32 of the base end side flexible tube 30. In contrast, the short traction wire 50A (53c, 54c, 55c and 56c) is formed one size longer than the length of the longitudinal direction L of the base end side flexible tube 30 in order to be arranged only in the base end side wire lumen 32 of the base end side flexible tube 30. In addition, an expanded diameter portion 51 is also provided at the distal end of the short traction wire 50A (53c, 54c, 55c and 56c).

The traction wire 50 and the short traction wire 50A are paired to form a set. Specifically, the traction wire 53a and the short traction wire 53c form a first wire set 53A, the traction wire 54a and the short traction wire 54c form a second wire set 54A, the traction wire 55a and the short traction wire 55c form a third wire set 55A, and the traction wire 56a and the short traction wire 56c form a fourth wire set 56A.

The assembly of the movable elongated structure 10A having the above-described components will be described below.

First, from the distal end side LF toward the base end side LB, the distal end side flexible tube 20A, the spacer 40A, and the base end side flexible tube 30 are arranged in series in this order along the longitudinal direction L. At this time, the distal end side flexible tube 20A and the base end side flexible tube 30 are arranged in a direction corresponding to the base end lumen set 32 (33 to 36) which is a pair of distal end side wire lumens 22A (23c to 26c) and base end side wire lumens 32 provided in four directions (up, down, left, and right).

Then, the traction wire 50 is inserted through the distal end side wire lumen 22A, the placement recess 44 and the base end side wire lumen 32, and the short traction wire 50A is inserted through the base end side wire lumen 32.

Specifically, in the distal end side flexible tube 20A, the traction wire 53a of the first wire set 53A is arranged in the distal end side wire lumen 23c in the upward direction HU among the four directions of the cross section in which the distal end side wire lumen 22A is provided. Then, in the base end side flexible tube 30, the traction wire 53a is arranged in the base end side wire lumen 36a among the base end side wire lumens 35b and 36a that are closer to the cross-sectional center "0" in the height direction H than the distal end side wire lumen 23c arranged in the distal end side flexible tube 20 and face each other in the width direction W, and the short traction wire 53c is arranged in the base end side wire lumen 35b .

In addition, in the spacer 40A arranged between the distal end side flexible tube 20 and the base end side flexible tube 30, the traction wire 53a is arranged in an appropriate arrangement recess 44 in the protruding edge portion 43 so that the traction wire 53a may be arranged as described above.

In addition, the expanded diameter portion 51 of the traction wire 53a is positioned and secured in the upward fixing recess 61 of the distal end cap 60 of the distal end side flexible tube 20 in the upward direction HU, and the expanded diameter portion 51 of the short traction wire 53c arranged in the base end side wire lumen 35b is fixed near the base end protrusion portion 43b of the spacer 40A.

In the distal end side flexible tube 20A, the traction wire 54a of the second wire set 54A is arranged in the distal end side wire lumen 24c in the downward direction HD among the four directions of the cross section in which the distal end side wire lumen 22A is provided. In the base end side flexible tube 30, the traction wire 54a is arranged in the base end side wire lumen 35a of the base end side wire lumens 35a and 36b that are closer to the cross-sectional center "0" in the height direction H than the distal end side wire lumen 24c arranged in the distal end side flexible tube 20 and face each other in the width direction W, and the short traction wire 54c is arranged in the base end side wire lumen 36b.

In addition, in the spacer 40A arranged between the distal end side flexible tube 20 and the base end side flexible tube 30, the traction wire 54a is arranged in an appropriate arrangement recess 44 in the protruding edge portion 43 so that the traction wire 54a may be arranged as described above.

In addition, the expanded diameter portion 51 of the traction wire 54a is positioned and secured in the fixed recess 61 in the downward direction HD of the distal end cap 60 of the distal end side flexible tube 20, and the expanded diameter portion 51 of the short traction wire 54c arranged in the base end side wire lumen 36b is fixed near the base end protrusion portion 43b of the spacer 40A.

In the distal end side flexible tube 20A, the traction wire 55a of the third wire set 55A is arranged in the distal end side wire lumen 25c on the right side WR of the four directions of the cross section in which the distal end side wire lumen 22A is provided. In the base end side flexible tube 30, the traction wire 55a is arranged in the base end side wire lumen 33a, which is closer to the cross-sectional center "0" in the width direction W than the distal end side wire lumen 25c arranged in the distal end side flexible tube 20, and is opposed to the cross-sectional center "0" in the height direction H. In addition, the short traction wire 55c is arranged in the base end side wire lumen 34b.

In addition, in the spacer 40A arranged between the distal end side flexible tube 20 and the base end side flexible tube 30, the traction wire 55a is arranged in an appropriate arrangement recess 44 in the protruding edge portion 43 so that the traction wire 55a may be arranged as described above.

In addition, the expanded portion 51 of the traction wire 55a is placed and secured in the fixed recess 61 on the right side WR of the distal end cap 60 of the distal end side flexible tube 20, and the expanded portion 51 of the short traction wire 55c arranged in the base wire lumen 34b is fixed near the base protrusion portion 43b of the spacer 40A.

In the distal end side flexible tube 20A, the traction wire 56a of the fourth wire set 56A is arranged in the distal end side wire lumen 26c on the left side WL among the four directions of the cross section in which the distal end side wire lumen 22A is provided. In the base end side flexible tube 30, the traction wire 56a is arranged in the base end side wire lumen 34a among the base end side wire lumens 33b and 34a that are closer to the cross-sectional center "0" in the width direction W than the distal end side wire lumen 26c arranged in the distal end side flexible tube 20 and face each other in the height direction H, and the short traction wire 56c is arranged in the base end side wire lumen 33b.

In addition, in the spacer 40A arranged between the distal end side flexible tube 20 and the base end side flexible tube 30, the traction wire 56a is arranged in an appropriate arrangement recess 44 in the protruding edge portion 43 so that the traction wire 56a may be arranged as described above.

In addition, the expanded portion 51 of the traction wire 56a is positioned and fastened in the fixed recess 61 on the left side WL of the distal end cap 60 of the distal end side flexible tube 20, and the expanded portion 51 of the short traction wire 56c arranged in the base end side wire lumen 33b is positioned and fastened in the vicinity of the base end protrusion portion 43b of the spacer 40A.

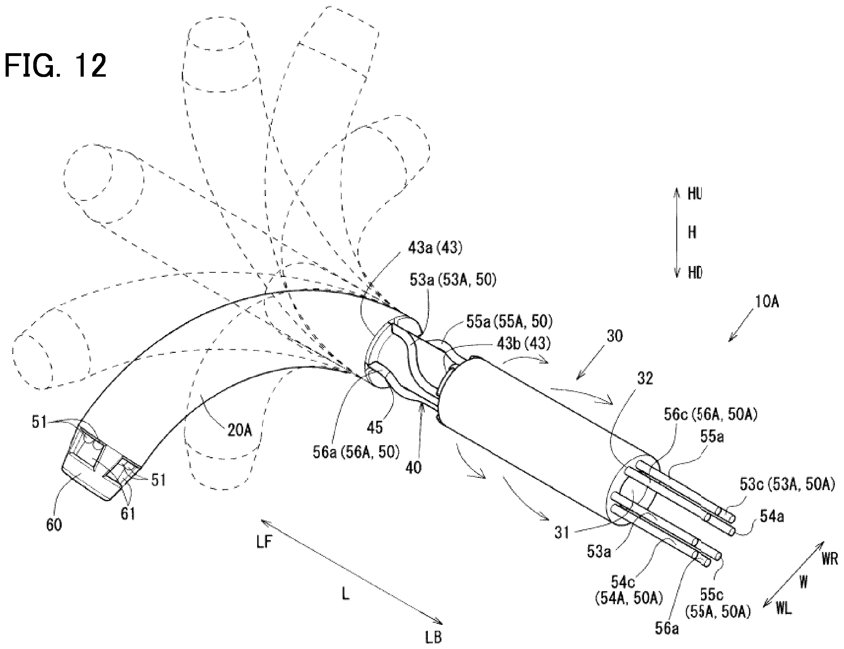

As shown in Fig. 12, the movable elongated structure 10A configured in this manner may smoothly bend and deform the distal end side flexible tube 20A relative to the base end side flexible tube 30 by towing the wire set (53A to 56A) consisting of a pair of the traction wire 50 and the short traction wire 50A toward the base end side LB. Furthermore, the movable elongated structure 10A may smoothly bend and deform the base end side flexible tube 30 in the longitudinal direction L by towing the short traction wire 50A toward the base end side LB, as shown by the arrow in Fig. 12.

Specifically, the distal end side flexible tube 20A may be bent in the upward direction HU by towing a first wire group 53A including a traction wire 53a arranged in the distal end side wire lumen 23c in the upward direction HU toward the base end side LB, and the distal end side flexible tube 20A may be bent in the downward direction HD by towing a second wire group 54A including a traction wire 54a arranged in the distal end side wire lumen 24c in the downward direction HD toward the base end side LB.

In the distal end side flexible tube 20A, the third wire group 55A including the traction wire 55a arranged in the distal end side wire lumen 25c of the right side WR is towed toward the base end LB, thereby allowing the distal end side flexible tube 20A to be bent and deformed to the right side WR. Furthermore, the fourth wire group 56A including the traction wire 56a arranged in the distal end side wire lumen 26c of the left side WL is towed toward the base end LB, thereby allowing the distal end side flexible tube 20A to be bent and deformed to the left side WL.

In addition, by towing the short traction wire 53c arranged in the base end side wire lumen 35b of the right side WR in the base end side flexible tube 30 toward the base end LB, the base end side flexible tube 30 may be bent and deformed to the right side WR, and by towing the short traction wire 54c arranged in the base end side wire lumen 36b of the left side WL toward the base end LB, the base end side flexible tube 30 may be bent and deformed to the left side WL.

Furthermore, by towing the short traction wire 55c arranged in the base end side wire lumen 34b in the downward direction HD of the base end side flexible tube 30 toward the base-end side LB, the base end side flexible tube 30 may be bent in the downward direction HD, and by towing the short traction wire 56c arranged in the base end side wire lumen 33b in the upward direction HU toward the base-end side LB, the base end side flexible tube 30 may be bent in the upward direction HU.

In this way, when the traction wire 50 and the short traction wire 50A are towed independently toward the base end side LB, it is possible to bend and deform one of the distal end side flexible tube 20A and the base end side flexible tube 30. When the traction wire 50 is towed toward the base end side LB, the distal end side flexible tube 20A and the base end side flexible tube 30 may be bent and deformed in four directions (up, down, left, and right directions) that are mutually perpendicular.

Of course, in the movable elongated structure 10A, the distal end side flexible tube 20A and the base end side flexible tube 30 may be bent and deformed in the desired direction by using multiple traction wires 50, multiple short traction wires 50A, or a combination of these.

As described above, in the movable elongated structure 10A, the long traction bodies (53a to 56a) of the wire set (53A to 56A) are arranged in the distal end side flexible tube (20) in one of the four directions (upward direction HU, downward direction HD, right side WR, left side WL) of the cross section in which the distal end side wire lumen 22A (23c to 26c) is provided, and are fixed at the distal end side (61) of the distal end side flexible tube (20) . In the base end side flexible tube (30), a short traction body (50A) is arranged in the base end side wire lumen (33a to 36a, 33b to 36b) which is closer to the cross-sectional center "0" in one direction than the distal end side wire lumen 22A (23c to 26c) arranged in the distal end side flexible tube (20) and faces the base end side wire lumen (33a to 36a, 33b to 36b) in a direction intersecting the one direction, and the short traction body (50A) arranged in the base end side wire lumen (33a to 36a, 33b to 36b) of the base end side flexible tube (30) is fastened to the base end side tubular body on the distal end side of the base end side flexible tube (30). Therefore, each of the traction bodies (50, 50A) is configured to be capable of being towed independently, and by traction the multiple traction bodies (50, 50A) toward the base end side (LB), at least one of the base end side flexible tube (30) and the distal end tubular body (20, 20A) may be bent and deformed in a desired direction relative to the other.

In addition, the traction wire 50 and the short traction wire 50A may be configured as a single long traction wire bent back at the base end side FB.

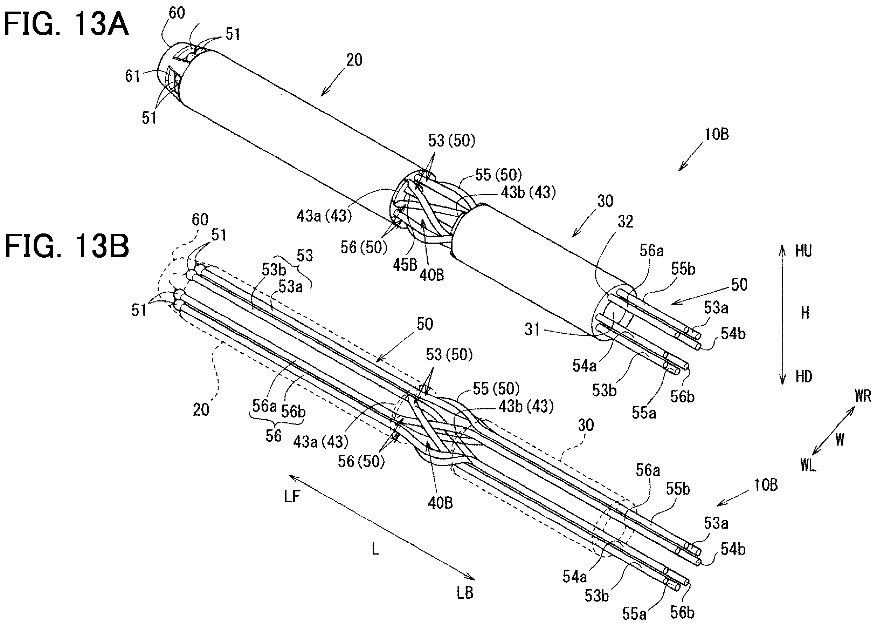

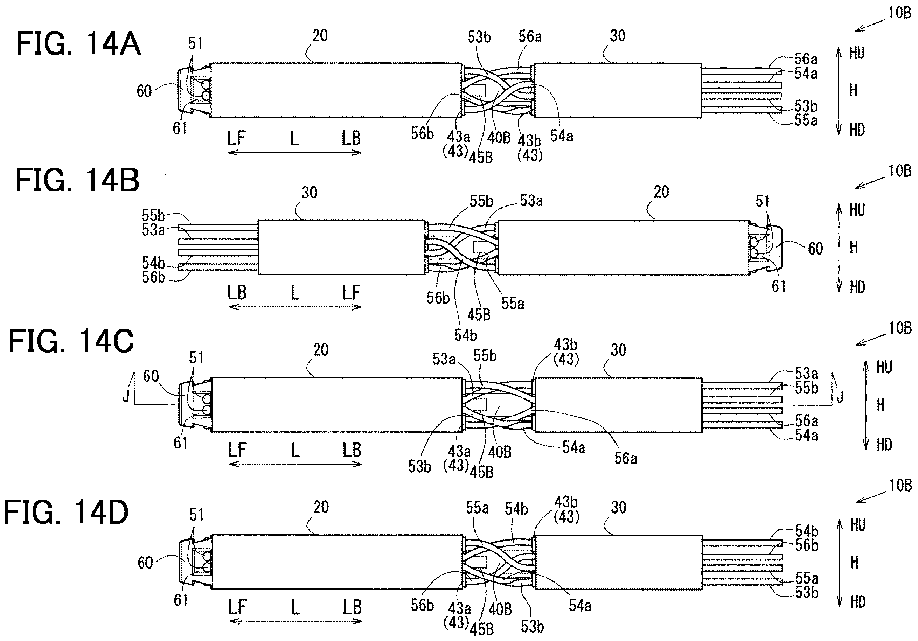

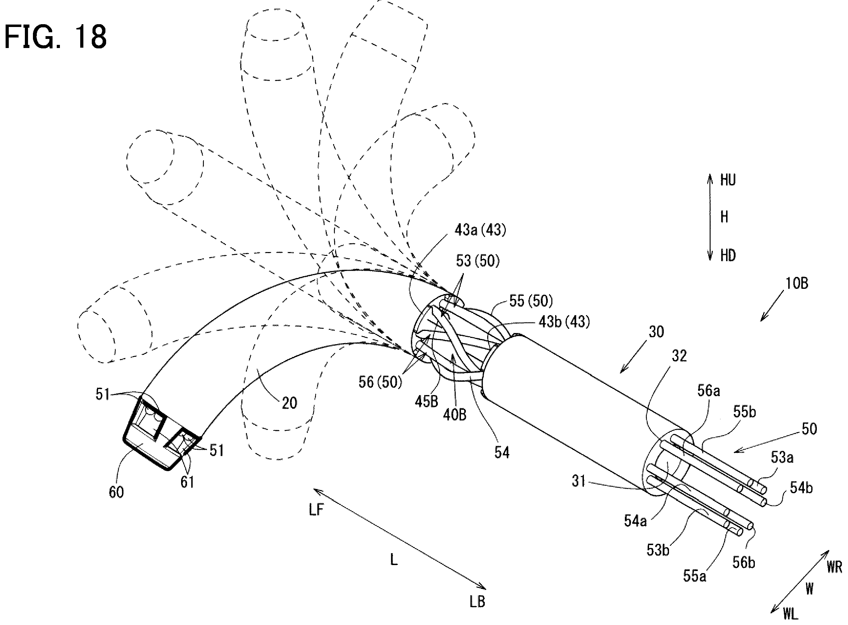

Next, a movable elongated structure 10B according to a different embodiment will be described with reference to FIGS. 13 to 18.

The movable elongated structure 10B has a spacer 40B which is different from the spacer 40 in the movable elongated structure 10 described above, but the other elements have the same configuration, and in addition, the routing path of the base end side wire lumen 32 in the base end side flexible tube 30 of the wire group (53 to 56) routed in the distal end wire lumen 22 (23 to 26) of the distal end flexible tube (20) is different.

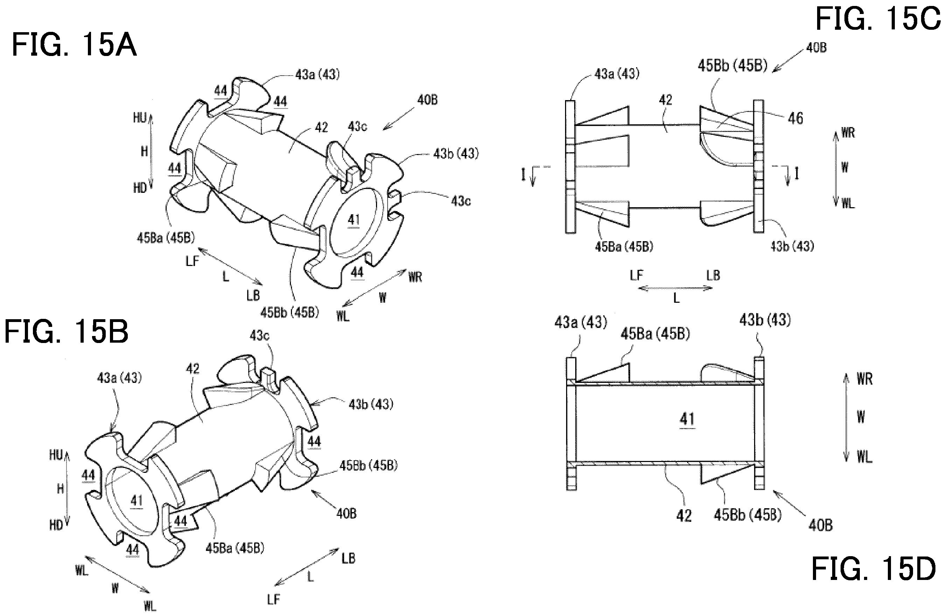

As shown in FIG. 15, the spacer 40B, like the above-mentioned spacer 40, is provided with a distal end side protruding edge portion 43a (43) having an arrangement recess 44 on the distal end side LF of a cylinder 42 having an internal space 41 therein, and a base end side protruding edge portion 43b (43) on the base end side LB. Note that the arrangement recess 44 on the upper direction HU and the right side WR of the base end side protruding edge portion 43b is provided with a partitioning protrusion 43c that partitions the traction wire 50 to be arranged.

Further, at the distal end side protruding edge portion 43a of the cylinder 42, at a position corresponding to the arrangement recess 44, there is provided a guide protrusion 45Ba whose protruding height gradually increases toward the base end side LB.

The base end side protruding edge 43b of the cylinder 42 is provided with a guide protrusion 45Bb whose protruding height gradually increases toward the base end side LB at a position corresponding to between the arrangement recesses 44 adjacent in the circumferential direction. The guide protrusion 45Bb has a groove 46 through which the traction wire 50 passes and is formed in a shape curved toward the circumferential direction. Since the guide protrusion 45Bb has the groove 46, the crossing traction wires 53b and 54a and the traction wires 55a 56b may be arranged so as not to interfere with each other, as described later.

In the above-mentioned movable elongated structure 10, in the distal end side flexible tube 20, the distal lumen set (23 to 26) is arranged in one of the four directions (upward direction HU, downward direction HD, right side WR, left side WL) of the cross section in which the distal end side flexible tube 20 has the distal lumen set (23 to 26), and in the base end side flexible tube 30, the distal lumen set (23 to 26) is arranged in the base end side wire lumen (33a to 36a, 33b to 36b) that is closer to the cross-sectional center "0" in one direction (upward direction HU, downward direction HD, right side WR, left side WL) than the distal lumen set (23 to 26) arranged in the distal end side flexible tube 20, and the distance between the arranged traction wires (53a to 56a, 53b to 56b) is wider.

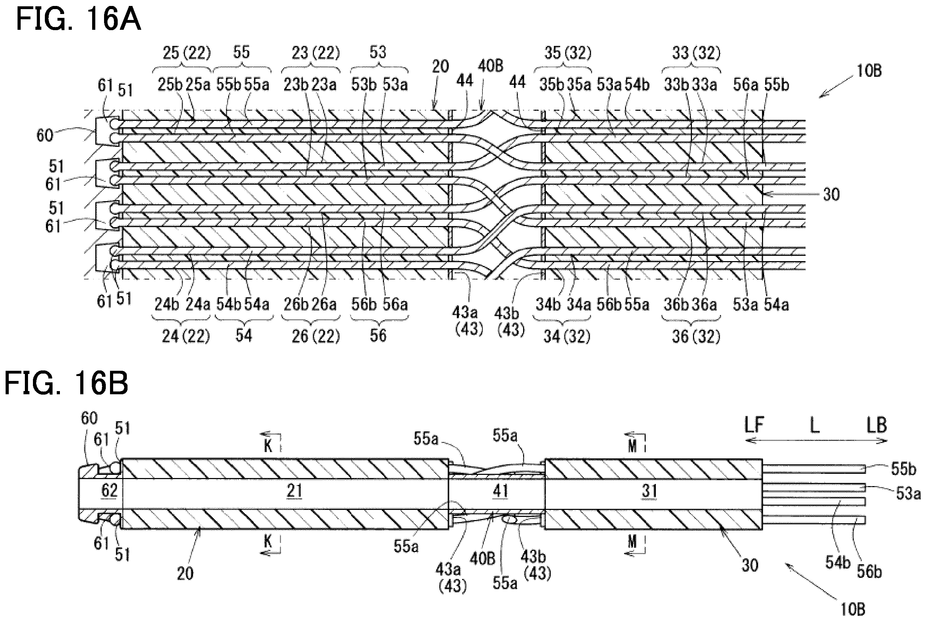

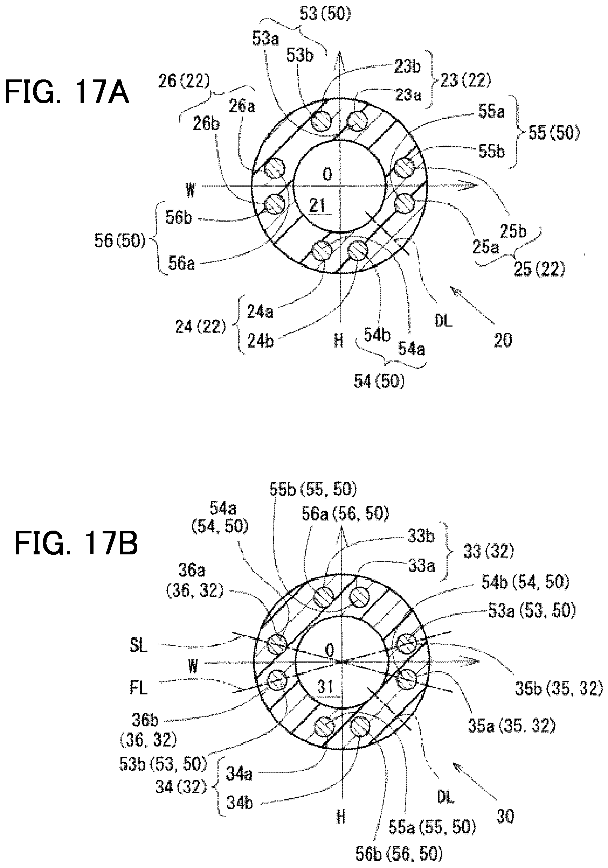

In contrast, in the movable elongated structure 10B, as shown in FIGS. 16A, 17A and 17B, in the base end side flexible tube 30, the distance to the cross-sectional center "0" in one direction (upward direction HU, downward direction HD, right side WR, left side WL) is closer than the distal end lumen set (23 to 26) arranged in the distal end flexible tube 20, and the distance between the arranged traction wires (53a to 56a, 53b to 56b) is wider.

Furthermore, in the movable elongated structure 10B, in the distal end side flexible tube 20, two sets of wires (53 to 56) are arranged in two sets of distal lumen sets (23 to 26) that face each other in one direction with the cross-sectional center "0" in between.

Then, in the base end side flexible tube 30, the traction wires (53a to 56a, 53b to 56b) in the two wire sets (53 to 56) are arranged in one of the base end side wire lumens (33a to 36a, 33b to 36b) in the two base-end lumen sets (33 to 36) that face each other across the cross-sectional center "0" in a crossing direction that intersects with one direction.

At this time, as shown in FIG. 17B, in the base end side flexible tube 30, a first virtual line FL connecting the traction wires of one of the two wire sets (53 to 56) arranged in any of the base end side wire lumens (33a to 36a, 33b to 36b) in the two lumen sets 33, 34, 35, and 36 in the cross-sectional direction and a second virtual line SL connecting the traction wires of the other wire set in the cross-sectional direction are arranged so as to intersect in the cross-sectional direction.

Specifically, in the distal end side flexible tube 20, a first wire set 53 is arranged in the distal end upper lumen set 23 in the upward direction HU of the four directions of the cross section in which the distal end side wire lumen 22 is provided, and a second wire set 54 is arranged in the distal end lower lumen set 24 in the downward direction HD.

The lumen sets 23 and 24 are arranged in the base end side flexible tube 30 in a width direction W perpendicular to the height direction H in cross section, and in lumen sets 35 and 36 having a wider gap therebetween than the gap between the lumen sets 23 and 24 in the width direction W.

One of the traction wires 53a and 53b arranged in the upper lumen set 23 on the distal end side in the upward direction HU is arranged in the wire lumen that is farther in the height direction H of the lumen sets 35 and 36, i.e., on the negative side of the H coordinate, and the other is arranged in the wire lumen that is closer in the height direction H of the lumen sets 35 and 36, i.e., on the positive side of the H coordinate.

In contrast, one of the traction wires 54a and 54b arranged in the lower lumen set 24 on the distal end side in the downward direction HD is arranged in the wire lumen that is farther in the height direction H of the lumen sets 35 and 36, i.e., on the +(plus) side of the H coordinate, and the other is arranged in the wire lumen that is closer in the height direction H of the lumen sets 35 and 36, i.e., on the -(minus) side of the H coordinate.

As an example, the traction wire 53a of the first wire set 53 arranged in the distal end side wire lumen 23a on the H coordinate +side and W coordinate +side of the distal end upper lumen set 23 of the distal end side flexible tube 20 is arranged in the base wire lumen 35b on the H coordinate +side and W coordinate +side of the lumen sets 35 and 36 in the base end side flexible tube 30, which is close in the height direction H.

In contrast, the traction wire 53b of the first wire set 53, which is arranged in the distal end side wire lumen 23b on the +H coordinate side and -W coordinate side of the distal end upper lumen set 23 of the distal end side flexible tube 20, is arranged in the base wire lumen 36b on the -H coordinate side and -W coordinate side of the lumen sets 35 and 36 in the base end side flexible tube 30, which is farther in the height direction H than the base wire lumen 36b on the -H coordinate side and -W coordinate side.

In addition, the traction wire 54b of the second wire set 54 arranged in the distal end side wire lumen 24b on the -H coordinate side and +W coordinate side of the distal end lower lumen set 24 of the distal end side flexible tube 20 is arranged in the base wire lumen 35a on the -H coordinate side and +W coordinate side of the lumen sets 35 and 36 in the base end side flexible tube 30, which is closer in the height direction H than the base wire lumen 35a on the -H coordinate side and +W coordinate side.

In contrast, the traction wire 54a of the second wire set 54 arranged in the distal end side wire lumen 24a on the -H coordinate side and -W coordinate side of the distal end lower lumen set 24 of the distal end side flexible tube 20 is arranged in the base wire lumen 36a on the +H coordinate side and -W coordinate side, which is farther in the height direction H of the lumen sets 35 and 36 in the base end side flexible tube 30.

In this way, the traction wire 53b arranged in the base end side wire lumen 36b far from the distal end side flexible tube 20 in the lumen sets 23 and 24 facing each other in the height direction H and the traction wire 54a arranged in the base end side wire lumen 36a among the wire sets 53 and 54 arranged in the lumen sets 35 and 36 facing each other in the width direction W in the base end side flexible tube 30 cross each other in the spacer 40B. However, the traction wire 53b and the traction wire 54a may be smoothly operated by towing to the base end side LB without interfering with each other due to the guiding convex portion 45B (45Ba and 45Bb) of the spacer 40B.

In the base end side flexible tube 30, the first virtual line FL connecting the traction wire 53a arranged in the base end wire lumen 35b on the H coordinate +side and the W coordinate +side and the traction wire 53b arranged in the base end side wire lumen 36b on the H coordinate -side and the W coordinate -side passes through the cross-sectional center "0", and connects the H coordinate -side and the W coordinate -side with the H coordinate +side and the W coordinate +side, and is in the upward right direction in FIG. 17 B.

In contrast, in the base end side flexible tube 30, the second virtual line SL connecting the traction wire 54b arranged in the base end side wire lumen 35a on the H coordinate -side and the W coordinate +side and the traction wire 54a arranged in the base end side wire lumen 36a on the H coordinate +side and the W coordinate -side passes through the cross-sectional center "0", and connects the H coordinate +side and the W coordinate -side and the H coordinate -side and the W coordinate +side, and is in a right-downward direction in FIG. 17B.

Therefore, the first virtual line FL passing through the traction wires 53a and 53b and the second virtual line SL passing through the traction wires 54a and 54b intersect at the cross-sectional center "0", as shown in FIG. 17B.

Similarly, in the distal end side flexible tube 20, a third wire set 55 is arranged in the distal right lumen set 25 on the right side WR of the four directions of the cross section in which the distal end side wire lumen 22 is provided, and a fourth wire set 56 is arranged in the distal left lumen set 26 on the left side WL.

The lumen sets 25 and 26 are arranged in the base end side flexible tube 30, facing each other in a height direction H perpendicular to the width direction W in cross section, and arranged in lumen sets 33 and 34 having a wider gap therebetween than the gap between the lumen sets 25 and 26 in the height direction H.

One of the traction wires 55b and 55a arranged in the right lumen set 25 on the distal end side of the right side WR is arranged in the wire lumen that is farther in the width direction W of the lumen sets 33 and 34, i.e., the negative side of the W coordinate, and the other is arranged in the wire lumen that is closer in the width direction W of the lumen sets 33 and 34, i.e., the positive side of the W coordinate.

In contrast, one of the traction wires 56b and 56a arranged in the left lumen set 26 on the distal end side of the left side WL is arranged in the wire lumen that is farther in the width direction W of the lumen sets 33 and 34, i.e., the +side of the W coordinate, and the other is arranged in the wire lumen that is closer in the width direction W of the lumen sets 33 and 34, i.e., the -side of the W coordinate.

As an example, the traction wire 55b of the third wire set 55 arranged in the distal end right wire lumen 25b on the W coordinate +side and H coordinate +side of the distal end right lumen set 25 of the distal end side flexible tube 20 is arranged in the base wire lumen 33a on the W coordinate +side and H coordinate +side of the lumen sets 33 and 34 in the base end side flexible tube 30, which is closer in the width direction W.

In contrast, the traction wire 55a of the third wire group 55, which is arranged in the distal end right wire lumen 25a on the +W coordinate side and the -H coordinate side of the distal end right lumen group 25 of the distal end side flexible tube 20, is arranged in the base wire lumen 34a on the -W coordinate side and the -H coordinate side, which is farther in the width direction W of the lumen groups 33 and 34 in the base end side flexible tube 30.

In addition, the traction wire 56a of the fourth wire set 56, which is arranged in the distal end side wire lumen 26a on the -side of the W coordinate and the +side of the H coordinate of the distal end left lumen set 26 of the distal end side flexible tube 20, is arranged in the base wire lumen 33b on the -side of the W coordinate and the +side of the H coordinate, which is closer in the width direction W of the lumen sets 33 and 34 in the base end side flexible tube 30.

In contrast, the traction wire 56b of the fourth wire group 56, which is arranged in the distal end side wire lumen 26b on the -side of the W coordinate and the -side of the H coordinate of the distal end left lumen group 26 of the distal end side flexible tube 20, is arranged in the base wire lumen 34b on the + side of the W coordinate and the -side of the H coordinate, which is farther in the width direction W of the lumen groups 33 and 34 in the base end side flexible tube 30.

In this way, the traction wire 55a arranged in the base end side wire lumen 34a far from the distal end side flexible tube 20 in the lumen sets 25 and 26 facing each other in the height direction H in the base end side flexible tube 30, and the traction wire 56b arranged in the base end side wire lumen 34b, among the wire sets 55 and 56 arranged in the lumen sets 33 and 34 facing each other in the width direction W in the base end side flexible tube 30, cross each other in the spacer 40B. However, the guiding convex portion 45B of the spacer 40B allows the traction wire 55a and the traction wire 56b to operate smoothly by towing to the base end side LB without interfering with each other.

In the base end side flexible tube 30, the first virtual line FL connecting the traction wire 55b arranged in the base end side wire lumen 33a on the W coordinate +side and the H coordinate +side and the traction wire 55a arranged in the base end side wire lumen 34a on the W coordinate -side and the H coordinate -side passes through the cross-sectional center "0", and connects the W coordinate -side and the H coordinate -side with the W coordinate +side and the H coordinate +side, and is in the upward right direction in FIG. 17 B.

In contrast, in the base end side flexible tube 30, the second virtual line SL connecting the traction wire 56a arranged in the base end side wire lumen 33b on the W coordinate -side and the H coordinate +side and the traction wire 56b arranged in the base end side wire lumen 34b on the W coordinate +side and the H coordinate -side passes through the cross-sectional center "0", and connects the W coordinate +side and the H coordinate -side and the W coordinate -side and the H coordinate +side, and is in the downward right direction in FIG. 17 B.

Therefore, the first virtual line FL passing through the traction wires 55b, 55a and the second virtual line SL passing through the traction wires 56b and 56a intersect at the cross-sectional center "0", as shown in FIG. 17B.

In the movable elongated structure 10B configured in this manner, by towing any one of the wire groups (53 to 56) or a combination of these toward the base end LB, the distal end side flexible tube 20 may be bent and deformed in the desired direction relative to the base end side flexible tube 30.

As described above, the first virtual line FL and the second virtual line SL based on the routing paths of the traction wires 50 in two of the four wire pairs (53 to 56) both pass through the cross-sectional center "0" in the base end side flexible tube 30 and intersect at the cross-sectional center "0". Therefore , with respect to the direction in which the distal end flexible tube 20 is bent by towing any of the four wire pairs (53 to 56), the towing force is not eccentric in the relevant direction with respect to the base end side flexible tube 30, and therefore it is possible to almost completely prevent the base end side flexible tube 30 from bending in the relevant direction.

The movable elongated structure 10B thus configured includes a plurality of flexible tubes (20 and 30) each having an internal space (21 and 31) penetrating therethrough in the longitudinal direction (L), a long, flexible traction wire (50), and a wire (50) arranged between two adjacent flexible tubes (20 and 30) in the longitudinal direction (L) among the plurality of flexible tubes (20 and 30) arranged in series to maintain a distance between the opposing ends of the flexible tubes (20 and 30). and a wire assisting tool (40B), in which the direction in which the traction wire (50) is towed in the longitudinal direction (L) is the base end side (LB) and the opposite side is the distal end side (LF), and among two flexible tubes (20 and 30) adjacent to each other in the longitudinal direction (L) in a plurality of flexible tubes (20 and 30) arranged in series, the flexible tube (20 and 30) arranged on the base end side (LB) is a base end side flexible tube (30), and the flexible tube (20 and 30) arranged on the distal end side (LF) is a distal end side flexible tube (20), a pair of traction wires (50) are a wire set (53 and 54), and two sets of wire sets (53 and 54) are provided, a wire lumen (22 and 32) for arranging the traction wire (50) along the longitudinal direction (L) is provided in the flexible tube (20 and 30), and the pair of wire lumens (22 and 32) are a lumen set (23, 24, 35 and 36), and two sets of lumen sets (23, 24, 35 and 36) are provided in the flexible tube (20 and 30), and the two sets of lumen sets (23, 24, 35 and 36) are arranged at opposing locations in the cross section of the flexible tubes (20 and 30), wherein the distal end side flexible tube (20) is arranged in series with the base end side flexible tube (30) in a direction in which the opposing directions in which the lumen sets (23, 24, 35 and 36) in the distal end side flexible tube (20) are arranged intersect with the opposing directions in which the lumen sets (23 and 24, 35 and 36) in the base end side flexible tube (30) are arranged.

The two wire sets (53 to 56) are arranged in the distal end side flexible tube (20) in the two lumen sets (23, 24, 35 and 36), respectively, and in the base end side flexible tube (30), the traction wire (50) of one of the two wire sets (53, 54) is arranged in one of the wire lumens (22 and 32) in the two lumen sets (23, 24, 35, 36), respectively, and the traction wire (50) of the other wire set (53 and 54) is arranged in one of the two lumen sets (23, 24, 35 and 36), respectively, wherein in the base end side flexible tube (30), the traction wires (50) in the two wire sets (53 and 54) are arranged in either of the wire lumens (22 and 32) in the two lumen sets (23, 24, 35 and 36) that face each other across the cross-sectional center "0" of the flexible tube (20 and 30) in a cross direction that crosses one direction, and in the base end side flexible tube (30), the traction wires (50) in the two lumen sets (35 and 36) are arranged in either of the wire lumens (22 and 32) in the two lumen sets (35 and 36), wherein a first virtual line (FL) connecting the traction wires (50) of one of the wire sets (53 to 56) of the two wire sets (53 and 54) arranged in the distal end 35a, 36a, 35b and 36b in the cross-sectional direction and a second virtual line (SL) connecting the traction wires (50) of the other wire set (53, 54) in the cross-sectional direction intersect in the cross-sectional direction, and wherein by towing the multiple traction wires (50) toward the base end side (LB) , at least one of the base end side flexible tube (30) and the distal end side flexible tube (20) may be curved and deformed in a desired direction relative to the other .

The movable elongated structure 10B may be configured with two wire sets. In this case, the distal end side flexible tube 20 has two lumen sets arranged at opposing positions in the cross section. For example, the distal end side flexible tube 20 may include lumen sets 23 and 24 and wire sets 53 and 54, and the distal end side flexible tube 20 may have the wire sets 53 and 54 wired to the lumen sets 23 and 24 as described above. Then, in the base end side flexible tube 30, the wire sets 53, 54 are wired to the lumen sets 35 and 36 as described above.

In this way, the movable elongated structure 10B has two lumen sets in the distal end side flexible tube 20 and two wire sets, and by towing one of the two wire sets toward the base-end side LB, the distal end side flexible tube 20 may be bent and deformed relative to the base end side flexible tube 30 in the direction of the lumen set to which the wire set is routed.

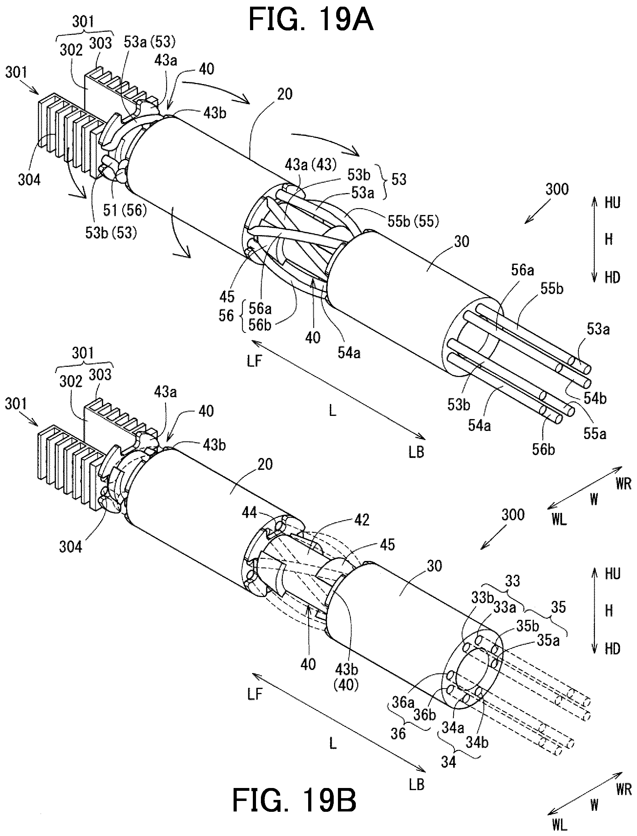

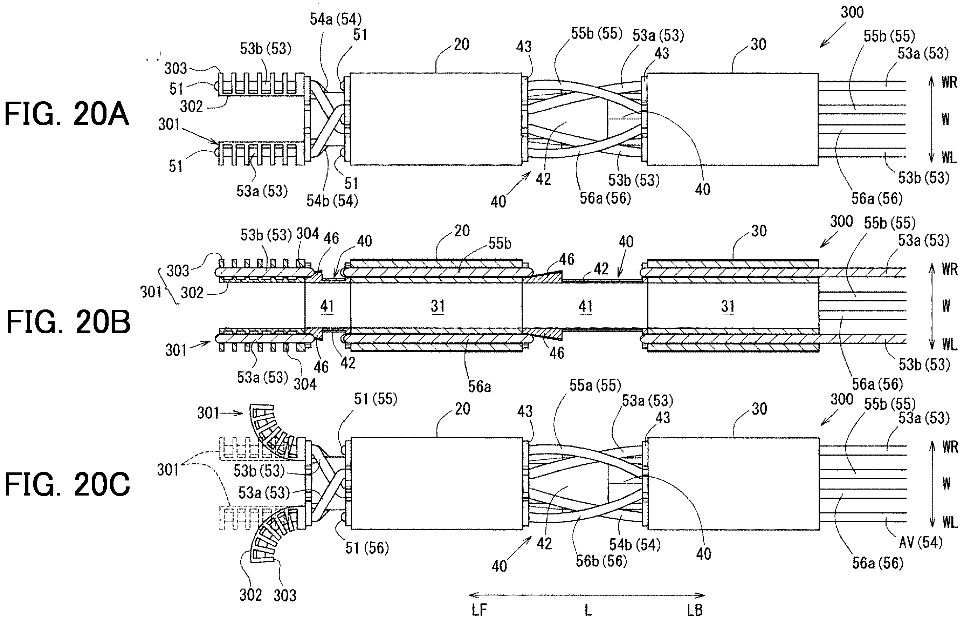

As shown in FIGS. 19 and 20, a retractor 300 may be configured using the movable elongated structure 10.

In the following description, the movable elongated structures 10, 10A and 10B are collectively referred to as the movable elongated structure 10. Therefore, in the following description, the description of the movable elongated structure 10 may be read as the movable elongated structures 10A and 10B. Similarly, the distal end side flexible tubes 20 and 20A are collectively referred to as the distal end side flexible tube 20, and in the following description, the description of the distal end side flexible tube 20 may be read as the distal end side flexible tube 20A.

FIGS. 19A and 19B show explanatory diagrams of a retractor 300. FIG. 19A shows a perspective view of the retractor 300, and FIG. 19B shows a perspective view of the retractor 300 in which the traction wire 50 is illustrated in a see-through state.

FIGS. 20A to 20C show schematic diagrams of the retractor 300. FIG. 20A shows a plan view of the retractor 300, FIG. 20B shows a cross section through the upper wire lumen, and FIG. 20C shows a plan view of the retractor 300 with the elastic retractor 301 in an open state.

The retractor 300 utilizes the above-mentioned movable elongated structure 10, and includes an elastic retractor 301 on the distal end side LF of the spacer 40, instead of the distal end side flexible tube 20 in the movable elongated structure 10. In more detail, the above-mentioned movable elongated structure 10 is used in an orientation in which the width direction W is equal to the height direction H.

The spacer 40 in the retractor 300 is formed to have a shorter length in the longitudinal direction L of the cylinder 42 than the spacer 40 on the distal end side LF in the movable elongated structure 10, but the other configurations are the same. The arrangement of the traction wire 50 is also the same as that in the movable elongated structure 10, and therefore will not be described.

The two elastic retractors 301 are arranged facing each other in the width direction W and fixed to the distal end side LF of the spacer 40.

The elastic retractor 301 is composed of an elastic body 302 extending toward the distal end side LF, and a plurality of plate portions 303 protruding outward in the width direction W from the elastic body 302.

The elastic body 302 is a rectangular plate having a predetermined thickness that is longer in the longitudinal direction L than in the height direction H, and the plate portion 303 is a rectangular plate having a predetermined thickness that is longer in the height direction H than in the width direction W.

A plurality of the plate portions 303 is arranged at predetermined intervals in the longitudinal direction L and are integrally formed with the elastic body 302 into an approximately rectangular parallelepiped shape that is longer in the height direction H than in the width direction W and longer in the longitudinal direction L than the height direction H.

The plate portions 303 are arranged at a predetermined interval in the longitudinal direction L, and have insertion holes 304 formed therein through which the wire sets 53 and 54 are inserted, corresponding to the lumen sets 33 and 34 of the distal end side flexible tube 20 in the movable elongated structure 10.

The elastic retractor 301 configured in this manner is positioned on the distal end side LF of the spacer 40, facing each other at a predetermined distance in the width direction W, i.e., so that the direction in which the plate portion 303 protrudes relative to the elastic main body 302 is on the outside of the width direction W.

The traction wires 53a and 54b are inserted through the insertion holes 304 of the elastic retractor 301 on the left side WL, and the traction wires 53b and 54a are inserted through the insertion holes 304 of the elastic retractor 301 on the right side WR.

The retractor 300 configured in this manner may bend and deform the distal end side flexible tube 20 in the width direction W, as shown by the arrow in FIG. 19 A, by towing the wire pairs 55 and 56 arranged in the height direction H at the base-end side LB toward the base-end side LB.

Specifically, the distal end side flexible tube 20 may be bent to the left side WL by towing the fourth wire group 56 inserted into the distal left lumen group 26 of the distal end side flexible tube 20 toward the base end side LB, and the distal end side flexible tube 20 may be bent to the right side WR by towing the third wire group 55 inserted into the distal right lumen group 25 of the distal end side flexible tube 20 toward the base end side LB.

Then, by releasing the towing on the third wire group 55 and the fourth wire group 56, the bending caused by the towing on the third wire group 55 and the fourth wire group 56 is eliminated by the restoring force due to the elasticity of the distal end side flexible tube 20.

Then, by towing the wire pair 53 and 54 on the base end side LB, the elastic retractor 301 may be put into an open state.

Specifically, by towing the traction wires 53a and 54b inserted through the insertion hole 304 of the elastic retractor 301 on the left side WL, the elastic retractor 301 on the left side WL may be bent and deformed toward the left side WL, which is outside the width direction W, as shown by the arrow in FIG. 19A without bending and deforming the distal end side flexible tube 20.

Conversely, by towing the traction wires 53b and 54a inserted through the insertion hole 304 of the elastic retractor 301 on the right side WR, the elastic retractor 301 on the right side WR may be bent and deformed toward the right side WR, which is outside the width direction W, as shown by the arrow in FIG. 19 A, without bending and deforming the distal end side flexible tube 20.

Therefore, when the first wire group 53 and the second wire group 54 are towed simultaneously, as shown in FIG. 20 C, the elastic retractors 301 on both sides in the width direction W are curved and deformed toward the outside in the width direction W, so that the retractor 300 may be used as a retractor for opening a specified area.

Furthermore, by releasing the traction on the first wire group 53 and/or the second wire group 54, the bending caused by the traction on the first wire group 53 and/or the second wire group 54 is eliminated by the elastic restoring force of the elastic retractor 301 (302).

Furthermore, in the above-mentioned retractor 300, the elastic retractor 301 is used in which a plate portion 303 is provided on the outside of the elastic body 302 in the width direction W. However, by using an elastic retractor 301 in which the elastic body 302 is disposed on the outside of the width direction W and the plate portion 303 is provided on the inside of the elastic body 302 in the width direction W, the elastic retractor 301 may be used as forceps in which the elastic retractor 301 is bent inward in the width direction W by towing the first wire group 53 and/or the second wire group 54.

The above-mentioned retractor 300 utilizes the above-mentioned movable elongated structure 10 and has an elastic retractor 301 on the distal end LF of the spacer 40 instead of the distal end side flexible tube 20 in the movable elongated structure 10. However, instead of replacing the distal end side flexible tube 20, a movable elongated treatment instrument may be provided with a retractor on the end of the distal end LF of the distal end side flexible tube 20 and a drive mechanism for the retractor in the main lumen 21 of the distal end side flexible tube 20. Also, as the movable elongated treatment instrument, instead of the retractor provided on the end of the distal end LF of the distal end side flexible tube 20, a treatment instrument such as a gripper, forceps, tweezers, needle, probe, or scissors may be provided, and a drive mechanism for the treatment instrument may be disposed in the main lumen 21. Also, in order to heat, cauterize, stop bleeding, or cut biological tissue with these treatment instruments, a cable for transmitting high frequency (radio wave) or microwaves connected to the treatment instrument may be wired.

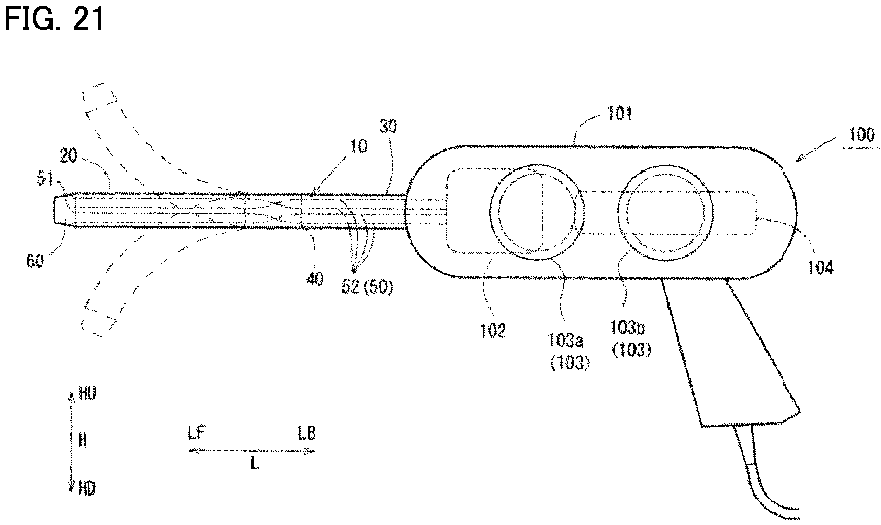

In the following, a manipulator 100, which is a medical device in another embodiment using the movable elongated structure 10 of the present invention, will be described, as shown in FIG. 21. Here, an example using a movable elongated structure 10 in which a distal end side flexible tube 20, a spacer 40, and a base end side flexible tube 30 are arranged in this order will be described, but the present invention is not limited to this. Note that FIG. 21 shows a schematic diagram of a manipulator 100, which is a medical device in another embodiment using the movable elongated structure 10 of the present invention.

The manipulator 100 is a medical instrument that is inserted into a duct having branching passages, such as inside a blood vessel, a lumen, or a hollow organ, and performs a specified treatment after the distal end reaches a specified location. It is equipped with a manipulator body 101 having a grip that is held by the surgeon, and a movable elongated structure 10 that extends from the distal end of the manipulator body 101 to the distal end side LF.

The manipulator body 101 is equipped with a traction drive unit 102 that traction wires 50 (not shown) extending from the base end side LB of the movable elongated structure 10, operating handles 103 (103a and 103b) that traction wires 50 with the traction drive unit 102 to manually control the bending direction of the movable elongated structure 10, and a control unit 104 that controls how the eight traction wires 50 are towed by the traction drive unit 102 by manually operating the operating handle 103.

The traction drive unit 102 and the control unit 104 are disposed inside the manipulator body 101, and the operating handle 103 is disposed outside the manipulator body 101.

The operation handle 103 includes a vertical operation handle 103a for bending the movable elongated structure 10 in the height direction H, and a width direction operation handle 103b for bending the movable elongated structure 10 in the width direction.

By manually operating the up-down operating handle 103a, the surgeon may bend the distal end side LF of the movable elongated structure 10 in the upward direction HU or downward direction HD in the height direction H, and may bend it by the desired amount depending on the amount of operation of the up-down operating handle 103a.

In addition, the surgeon may manually operate the width direction operating handle 103b to bend the flexible tube 20 at the distal end of the movable elongated structure 10 to the right or left in the width direction, and may bend the tube to the desired amount by adjusting the amount of operation of the width direction operating handle 103b.

The surgeon may manually operate both the up-down direction operating handle 103a and the width-direction operating handle 103b simultaneously or in sequence to bend and deform the distal end side LF of the movable elongated structure 10 in a diagonal direction intersecting the height direction H and the width direction, and may bend and deform in the desired bending direction in all directions depending on the amount of operation of the up-down direction operating handle 103a and the width-direction operating handle 103b.

In addition, the movable elongated structure 10 used in the above-mentioned manipulator 100 may be provided with a traction drive unit 102 corresponding to the direction of bending deformation, i.e., the number of traction wires 50, and an operating handle 103 corresponding to the direction of bending deformation.

If necessary, the operation handles 103a and 103b may be provided as separate units and connected to the manipulator body 101 by wire or wirelessly.

Furthermore, by making the length of the movable elongated structure 10 longer than the manipulator body 101 and inserting treatment instruments etc. into the main lumen 31 and internal space 41 as channels, the manipulator 100 may be used as a steering catheter that may be operated with the operating handle 103.

In addition, the length of the movable elongated structure 10 may be made longer than that of the manipulator body 101, and treatment instruments and the like may be inserted into the main lumen 31 and the internal space 41 as channels, for example, a camera may be mounted as an image receiving unit on the distal end side of the distal end side flexible tube 20 or on the distal end cap 60, so that the manipulator 100 may be used as a flexible endoscope that may be operated with the operating handle 103.

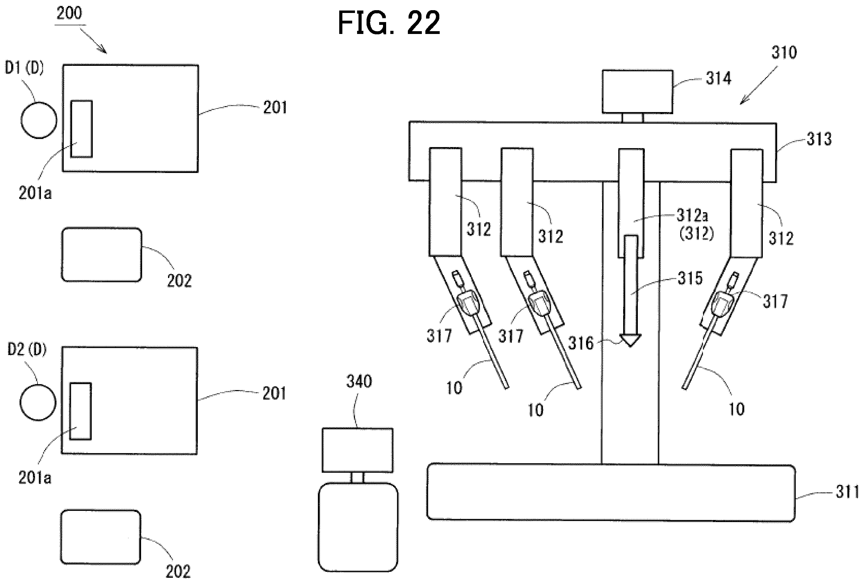

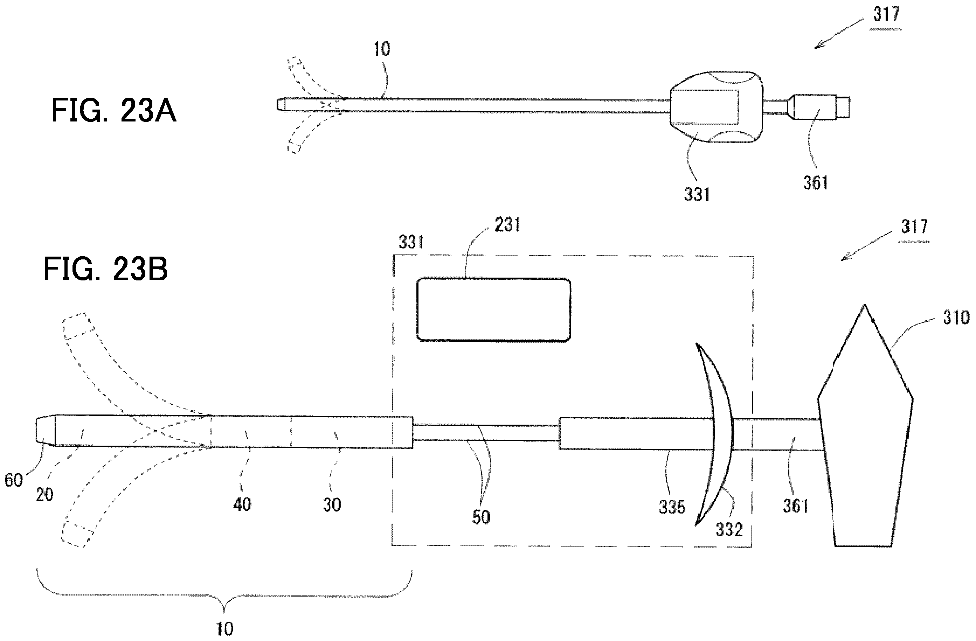

Next, a remote surgery system 200 according to one embodiment of the present invention will be described with reference to FIGS. 22 and 23.

Note that FIG. 22 is a schematic diagram of a remote surgery system 200 according to another embodiment, and FIG. 23 is a schematic diagram of a tool 317 in the remote surgery system 200. In detail, FIG. 23A shows a plan view of a tool 317 that may be mounted on a robot arm assembly of the remote surgery system 200, and FIG. 23B shows the internal configuration of the tool 317.

The telesurgery system 200 includes a surgeon console 201 serving as a station for each of two operators D (D1 and D2), a master control unit 202 operated by the operator D, a vision/core cart 340, and a patient side cart 310 robot.

The surgeon console 201 includes a viewer 201a in which an image of the surgical site is displayed to an operator D. When using the surgeon console 201, operators D1 and/or D2 typically sit in a chair at the surgeon console, position their eyes in front of the viewer 201a, and hold the master control unit 202 with one or both hands.

The remote surgery system 200 may be operated by two operators at the same time, but may also be operated by a single operator. When two operators operate simultaneously, they may cooperate with each other, which has the advantage of shortening the total surgery time for the patient. The system may be provided with three or more surgeon consoles 201 and master control units 202, if necessary.

The robot on the patient side cart 310 is located adjacent to the patient. During use, the patient side cart 310 is located near the patient requiring surgery. The robot on the patient side cart 310 is stationary during the surgical procedure but has casters on its base 311 to allow for mobility. The surgeon console 201 is used in the same operating room as the patient side cart, but may be located remotely from the patient side cart 310.

The patient side cart 310 includes four robot arm assemblies 312, but the number of the robot arm assemblies 312 is arbitrary. Each robot arm assembly 312 is connected to and driven and controlled by a drive unit 313 that enables three-dimensional movement.

A display 314 displays image data related to the procedure. The drive 313 is controlled by the master control unit 202 of the surgeon console 201. The movement of the tool 317 of the robotic arm assembly 312 is controlled by operation of the master control unit 202.

An image capture device 315, such as an endoscope, is disposed on one of the four robotic arm assemblies 312, robotic arm assembly 312a. The image capture device 315 includes a viewing camera 316 at its distal end. The image capture device 315 is an elongated shaft that allows the viewing camera 316 to be inserted through a surgical entry port in a patient (not shown).

The image capture device 315 is operatively connected to the viewer 201 a of the surgeon console 201 for displaying images captured by its viewing camera 316.

Each of the other robot arm assemblies 312 is a linkage device that supports and includes a detachable surgical instrument, a tool 317. If necessary, a visual camera may be mounted in a part of the main lumen 31 of the movable elongated structure 10 of the tool 317 of one or more robot arm assemblies 312 and used in place of the visual camera 316 of the image capture device 315.

The tool 317 includes an elongated movable elongated structure 10 that is elongated to allow insertion through a surgical entry port of a patient. The movement of the movable elongated structure 10 is controlled by the master control unit 202 of the surgeon console 201. The mobile elongate structure 10 utilizes the movable elongated structure 10 of the previous embodiments.

FIG. 23 shows a configuration of a tool 317 that may be loaded onto the robot arm assembly 312 of the telesurgery system 200 of FIG. 22 as a representative example of a surgical device. The tools 317 attached to the other robot arm assemblies 312 may have a similar configuration, or may be surgical devices of other configurations.

The tool 317 shown in FIG. 23A includes a movable elongated structure 10 having a traction wire 50, a surgical device 331 that drives, controls, and monitors the tool 317, and a connector 361 that connects to a robot. The surgical device 331 constitutes a towing drive unit that drives the traction wire 50 in the movable elongated structure 10.

As shown in FIG. 23 B, which illustrates the internal configuration of the tool 317, a medical system is shown that is composed of a surgical device 331 that drives the tool 317 directly connected to the robot arm assembly 312 in FIG. 22 via a shaft 335, and a robot on the patient side cart 310 that controls the surgical device 331.

The movable elongated structure 10 has a base end side flexible tube 30 connected to a shaft 335, a spacer 40, and a distal end flexible tube 20 which is an end effector. Since the movable elongated structure 10 has a bent structure as in the above-described embodiment, the freedom of the operating angle of the distal end flexible tube 20 is increased, improving the range of surgical application.

The surgical device 331 of the tool 317 has a control circuit 231 that controls signals within the surgical device and a signal interface 310 a with the robot of the patient side cart 310.

The control circuit 231 is configured to control a drive mechanism (not shown) that drives a predetermined traction wire 50 of the movable elongated structure 10 based on a control signal from the robot 310.

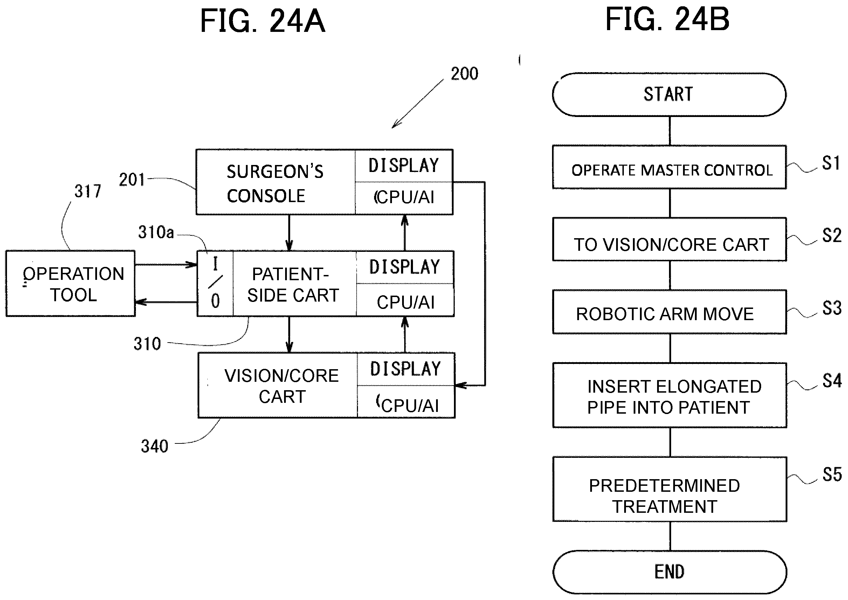

As shown in FIG. 24, the robot of the patient side cart 310 is connected to the surgical device 331 by wire and/or wirelessly via the signal interface 310a and the connector 361, and the robot of the patient side cart 310 is provided with an input unit that receives an operation signal from the master control unit 202, a calculation unit CPU that executes a predetermined operation program based on the operation signal , and an output unit that generates a drive signal for driving the movable elongated structure 10 of the tool 317 via the surgical device 331 based on the output from the calculation unit. The input unit and the output unit are composed of an input/output unit 310a (I/O).

FIGS. 24A and 24B are explanatory diagrams of a remote surgery system 200, in which FIG. 24A is a block diagram showing the connection relationships between each unit, and FIG. 24B is an operation flow diagram of the remote surgery system 200.

In the robot of the patient side cart 310 shown in FIG. 24, the arithmetic unit CPU that executes a predetermined operation program based on an operation signal may be equipped with artificial intelligence (Al). The robot of the patient side cart 310 equipped with artificial intelligence (Al) in the arithmetic unit CPU is provided with various sensors, accumulates and evaluates the detection results from the various sensors, and may control the tool 317 based on the evaluation results.

The vision and core cart 340 has a function related to the image capture device. When the remote surgery system 200 is started for surgery, the surgeon operates the master control unit 202 of the surgeon console 201, and in the case of two surgeons, the surgeon also operates the master control unit 202 of the surgeon console 201 (step S1), and the command generated by the operation is sent to the vision and core cart 340 (step S2).

The vision and core cart 340 then interprets the signals and moves the desired robotic arm assembly 312 to the patient's surgical area (step S3).

Next, the movable elongated structure 10 of the tool 317 attached to the selected robot arm assembly 312 is inserted into the patient through the long, thin pipe (step S4), and after reaching a predetermined location by bending the distal end side flexible tube 20, the predetermined treatment is performed (step S5), completing the surgery on the living tissue.

In addition, it is preferable to provide a traction drive unit 102 corresponding to the direction of bending deformation, i.e., the number of traction wires 50, and an operation handle 103 corresponding to the direction of bending deformation.

The remote surgery system 200 equipped with the above-mentioned manipulator 100, retractor 300, tool 317, and tool 317 is equipped with the movable elongated structure 10, and therefore may achieve the effects obtained by each configuration in addition to the effects provided by the above-mentioned movable elongated structure 10.

In the above, in correspondence between the configuration of the present invention and the above-mentioned embodiment, the longitudinal direction of the present invention corresponds to the longitudinal direction L, and similarly, the robot and the medical robot correspond to the patient side cart 310 , but are not limited to the above embodiment.

Although the traction wire 50 is used as the traction body, it may be in the form of a belt.

The distal end side flexible tube 20 is a tube having a circular ring-shaped cross section and an internal main lumen 31, but may be a cylindrica or tubular body having various ring-shaped cross sections such as an elliptical ring-shaped cross section, or a polygonal ring-shaped cross section such as a triangular or rectangular cross section, and the main lumen 31 may have a similar shape to the outer diameter of the cross-sectional shape as described above, or may have a different cross-sectional shape. Also, the main lumen 31 in the distal end side flexible tube 20 may be disposed at a position shifted from the center of the distal end side flexible tube 20.

In addition, the flexible tubes 20 and 30 may be configured with a backbone structure in which rigid bodies each having a main lumen therein are connected via a rotating joint or an elastic body.

Additionally, the telesurgery system 200 may be configured using a retractor 300 in place of the movable elongated structure 10 in the tool 317.

Furthermore, in the above explanation, flexible tubes 20 and 30 were used which are long cylindrical tubes having flexibility in the longitudinal direction L and having wire lumens 22 and 32 inside the tube wall, but flexible tubes made by weaving abrasion-resistant fibers, known as mesh tubes or braided tubes, may also be used.

In this case, a small-diameter tube constituting the main lumen 21 and 31 and a large-diameter tube constituting the outer shape are configured in two layers, and the wire lumen 22 and 32 is formed between the layers, while a flexible resin is filled in the gap. Even when a flexible tube configured by weaving abrasion-resistant fibers is used in this way, it is possible to achieve the same actions and effects as the movable elongated structure 10 using the flexible tubes 20 and 30 described above.

In addition, in the movable elongated structures 10 and 10B in which the traction wire 50 crosses the spacer 40, a spacer 40A (see FIGS. 9A to D) not having the guide convex portion 45 may be used. In this case, a coating or the like that improves the slipperiness may be applied to the surface of the traction wire 50. That is, in the movable elongated structures 10, 10B, instead of the guide convex portion 45 in the spacers 40, 40B, a coating or the like that improves the slipperiness may be applied to the surface of the traction wire 50, thereby achieving the same action and effect as the movable elongated structures 10, 10B using the spacers 40, 40B having the above-mentioned guide convex portion 45.

In addition, by chamfering the corners of the guiding protrusions 45 of the spacer 40, the traction wire 50 may be towed more smoothly.

Furthermore, the above-mentioned spacers 40, 40A, and 40B are configured by connecting the protruding edges 43 arranged at an interval in the longitudinal direction L with each other via a cylindrical cylinder 42.

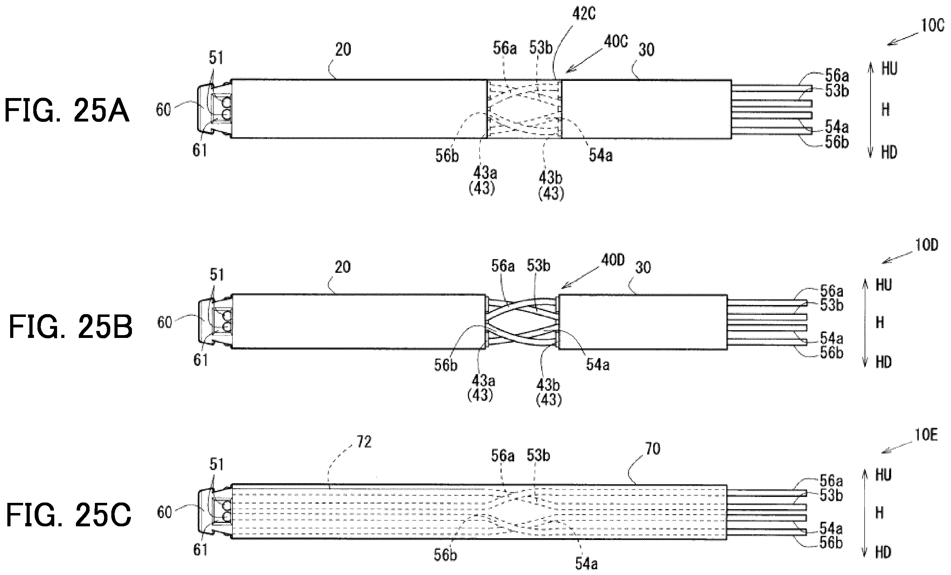

As shown in FIG. 25A, a spacer 40C may have a cylinder 42C formed along the outer edge of the protruding edge portion 43. In this way, the spacer 40C in which the protruding edge portions 43 arranged at intervals in the longitudinal direction L are connected to each other along the outer edge with a cylindrical cylinder 42C may accommodate the traction wire 50 passing between the distal end side flexible tube 20 and the base end side flexible tube 30 inside.

As described above, the movable elongated structure 10C using the spacer 40C may achieve the same functions and effects as the movable elongated structure 10 described above.

As shown in 25B, the spacer 40D may not include the cylinder 42. More specifically, the spacer 40D may be configured to include only the two protruding edge portions 43 spaced apart in the longitudinal direction L. In this case, the space between the protruding edge portions 43 spaced apart in the longitudinal direction L may be maintained by, for example, an exterior body surrounding the outside of the movable elongated structure 10D other than the spacer 40D.

As described above, the movable elongated structure 10D using the spacer 40D not including the cylinder 42 may achieve the same effects and advantages as the movable elongated structure 10 described above.

Furthermore, as shown in FIG. 25C, the movable elongated structure 10E may be configured without the spacer 40. In detail, the above-mentioned movable elongated structure 10 is configured by arranging the distal end side flexible tube 20, the spacer 40, and the base end side flexible tube 30 in this order from the distal end side LF toward the base end side LB, and wiring the traction wire 50 in the wire lumens 22, 32 of the distal end side flexible tube 20 and the base end side flexible tube 30. In contrast, as shown in FIG. 25C, the movable elongated structure 10E without the spacer 40 is provided with a main lumen (not shown) and a wire lumen 72 in a flexible tube 70 integrally formed with the distal end side flexible tube 20 and the base end side flexible tube 30. The wire lumen 72 is formed to pass through a path similar to the path in which the traction wire 50 is wired in the distal end side flexible tube 20, the spacer 40, and the base end side flexible tube 30 in the above-mentioned movable elongated structure 10. In this way, by inserting the traction wire 50 into the wire lumen 72 formed in the flexible tube 70 so as to pass through a path similar to the path along which the traction wire 50 is arranged in the movable elongated structure 10, the movable elongated structure 10E may achieve the same action and effect as the above-mentioned movable elongated structure 10.

Furthermore, in the above description, the wire lumens 22 and 32 are provided as through spaces inside the tube wall, and have flexibility in the form of a cylinder long in the longitudinal direction L, but the wire lumens 22 and 32 as through spaces may be replaced with grooves provided along the wire lumen, or with a restricting body that restricts the traction body to be wired from unintentionally coming off the wire lumen. In this way, by replacing the wire lumens 22 and 32 with grooves provided along the wire lumen, or with a restricting body that restricts the traction body to be wired from unintentionally coming off the wire lumen, it is possible to achieve the same effects and advantages as the movable elongated structure 10 using the flexible tubes 20, 30 having the wire lumens 22 and 32 as through spaces inside the wall.

In the above description, the enlarged diameter portion 51 of the traction wire 50, 50A is disposed and fixed at the distal end of the flexible tube 20, 30, so that the towing force for towing the traction wire 50, 50A acts on at least the flexible tube 20, 30. In contrast, the enlarged diameter portion 51 may not be provided on the traction wire 50, 50A, and the distal end of the traction wire 50, 50A may be fixed to the distal end of the flexible tube 20, 30 by a separate member such as adhesion or a clip. In addition, the distal ends of the traction wires 50, 50A adjacent to each other in the circumferential direction may be connected to each other and fixed by a separate member such as adhesion or a clip.

The traction wire 50 and the short traction wire 50A may be arranged in the base end side flexible tube 30 of the movable elongated structure 10A in the same manner as the traction wire 50 in the base end side flexible tube 30 of the movable elongated structure 10B.

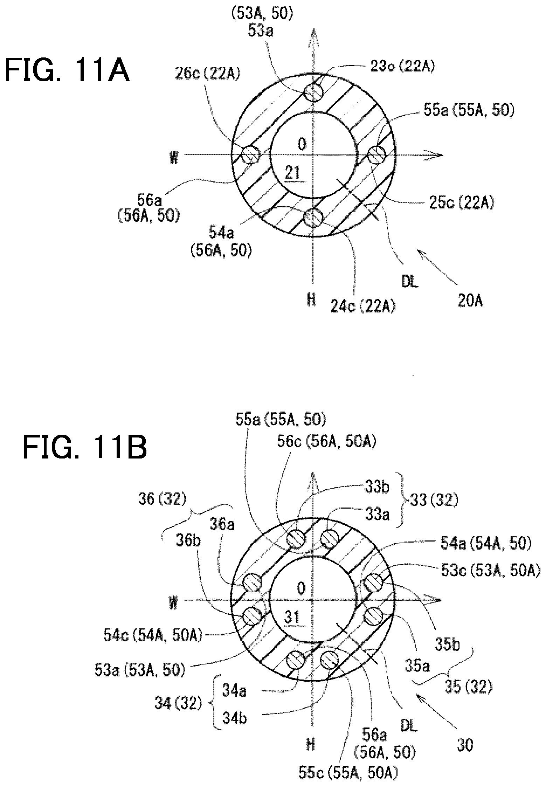

Specifically, in the base end side flexible tube, similarly to the above-mentioned movable elongated structure 10A (see FIG. 11), a traction wire 53a is inserted into the base end side wire lumen 36a, a short traction wire 54c is inserted into the base end side wire lumen 36b, a traction wire 56a is inserted into the base end side wire lumen 34a, and a short traction wire 55c is inserted into the base end side wire lumen 34b.

Then, in the base end side flexible tube, the short traction wire 54a is inserted into the base end side wire lumen 35a, the traction wire 53c is inserted into the base end side wire lumen 35b, the short traction wire 55a is inserted into the base end side wire lumen 33a, and the traction wire 56c is inserted into the base end side wire lumen 33b.

As a result, when a pair of traction wires consisting of the traction wire 50 and the short traction wire 50A is tied, the first virtual line FL and the second virtual line SL intersect in the cross-sectional direction as shown in FIG. 17B.

In the base end side flexible tube of the movable elongated structure 10A, the first virtual line FL and the second virtual line SL of the pair of traction wires, which are composed of the traction wire 50 and the short traction wire 50A arranged by the above-mentioned arrangement method, intersect so as to pass through the center of the cross section. Therefore, as described in the movable elongated structure 10B, the distal end flexible tube 20A may be bent in the desired direction by towing the traction wire without bending the base end side flexible tube.

### [Explanation of symbols]

10, 10A to 10E... Movable elongated structure
20, 20A... Distal end side flexible tube
21, 31...Main lumen
22, 32...Wire lumen
23, 24, 25, 26, 33, 34, 35, 36... Lumen set
30: Base end side flexible tube
40, 40A to 40D...Spacer
50, 50A...traction wire
53, 54, 55, 56...Wire set
L: Longitudinal direction
LB: Base end side
LF: Distal end side
FL: First virtual line
SL: Second virtual line
0 ... Center of section

## Claims

1. A movable elongated structure comprising:
a plurality of tubular bodies formed in a flexible, long shape and having an internal space penetrating in the longitudinal direction;
a flexible, elongated traction body; and
a wiring aid arranged between two of the tubular bodies arranged in series and adjacent to each other in the longitudinal direction, and maintaining a distance between the opposing ends of the tubular bodies;
wherein the direction in which the traction body is towed in the longitudinal direction is the base end side, and the opposite side is the distal end side;
wherein among the two tubular bodies adjacent to each other in the longitudinal direction in the plurality of tubular bodies arranged in series, the tubular body arranged on the base end side is the base end tubular body, and the tubular body arranged on the distal end side is a distal end side tubular body;
wherein a wire lumen is provided in the tubular body for wiring the traction body along the longitudinal direction; and
wherein the traction body is wired in the wiring aid at an angle with respect to the longitudinal direction, thereby allowing at least the distal end side tubular body to be curved and deformed in a desired direction.

2. The movable elongated structure according to claim 1,
wherein a pair of the traction bodies is a traction body set, and four sets of the traction body sets are provided,
wherein a pair of the wire lumens is a wire lumen set, and four sets of the wire lumen sets are provided,
wherein the four sets of the wire lumen sets are arranged in four directions in a cross section of the tubular body,
wherein the base end side tubular body and the distal end side tubular body arranged in series are arranged so that the wire lumen sets arranged in the four directions are oriented along the longitudinal direction,
wherein the traction body set is arranged in the wire lumen set in one of the four directions of the cross section in which the wire lumen set is arranged in the distal end side tubular body,
wherein the traction body set is arranged in the wire lumen in the base end side tubular body so that the distance to the cross-sectional center of the tubular body in the one direction is closer than the wire lumen set arranged in the distal end side tubular body, and the distance between the traction bodies arranged is wider,
wherein in the distal end side tubular body, the two sets of traction body sets are arranged in two sets of the wire lumen sets that face each other in one direction across the cross-sectional center of the tubular body,
wherein in the base end side tubular body, the traction bodies in the two sets of traction body sets are arranged in one of the wire lumens in the two sets of wire lumen sets that face each other across the cross-sectional center of the tubular body in a cross-sectional direction that crosses one direction,
wherein in the base end side tubular body, a first virtual line that connects the traction bodies of one of the two sets of traction body sets that are arranged in one of the wire lumens in the cross-sectional direction and a second virtual line that connects the traction bodies of the other of the traction body sets in the cross-sectional direction intersect in the cross-sectional direction, and
wherein by towing the multiple traction bodies toward the base end side, at least one of the base end side tubular body and the distal end and tubular body is curved and deformed in a desired direction relative to the other.

3. The movable elongated structure according to claim 1,
wherein a pair of the traction bodies is a traction body set, and two sets of the traction body sets are provided, in which a pair of the wire lumens is a wire lumen set,
wherein two sets of the wire lumen sets are provided in the tubular body, and the two sets of the wire lumen sets are arranged at opposing locations in the cross section of the tubular body,
wherein the distal end side tubular body is arranged in series with the base end side tubular body in a direction in which the opposing directions in which the wire lumen sets in the distal end side tubular body are arranged cross the opposing directions in which the wire lumen sets in the base end side tubular body are arranged,
wherein the two sets of the traction body sets are respectively arranged in the two sets of wire lumen sets in the distal end side tubular body,
wherein in the base end side tubular body, the traction bodies in the two traction body sets are arranged in one of the wire lumens in the two wire lumen sets that face each other across the cross sectional center of the tubular body in a cross-sectional direction that crosses one direction,
wherein in the base end side tubular body, a first virtual line connecting the traction bodies of one of the two traction body sets arranged in one of the wire lumens in the cross-sectional direction and a second virtual line connecting the traction bodies of the other traction body set in the cross-sectional direction intersect in the cross-sectional direction,
wherein by towing the plurality of traction bodies toward the base end side, at least one of the base end side tubular body and the distal end side tubular body is curved and deformed in a desired direction relative to the other.

4. The movable elongated structure according to claim 1,
wherein a pair of the traction bodies is a traction body set, and four sets of the traction body sets are provided,
wherein a pair of the wire lumens is a wire lumen set, and four sets of the wire lumen sets are provided,
wherein the four sets of the wire lumen sets are arranged in four directions in a cross section of the tubular body,
wherein the base end side tubular body and the distal end side tubular body arranged in series are arranged so that the wire lumen sets arranged in the four directions are oriented along the longitudinal direction,
wherein the traction body set is arranged in the wire lumen set in one of the four directions of the cross section in which the wire lumen set is arranged in the distal end side tubular body,
wherein the traction body set is arranged in the wire lumen in the base end side tubular body so that the distance to the cross-sectional center of the tubular body in the one direction is closer than the wire lumen set arranged in the distal end side tubular body, and the distance between the traction bodies arranged is wider,
wherein the traction bodies are arranged in the wire lumen of the base end side tubular body and the wire lumen of the distal end side tubular body, and are configured to be independently towed by being fastened to the distal end tubular body at the distal end side of the distal end side tubular body, and
wherein at least one of the base end side tubular body and the distal end side tubular body is curved and deformed in a desired direction relative to the other by towing the plurality of traction bodies toward the base end side.

5. The movable elongated structure according to claim 1,
wherein pair of the traction bodies is a traction body set, and four sets of the traction body sets are provided,
wherein one of the pair of traction bodies is a long traction body that is routed across the base end side tubular body and the distal end side tubular body, and the other is a short traction body that is routed to the base end side tubular body,
wherein a pair of the wire lumens is a wire lumen set, and four sets of the wire lumen sets are provided,
wherein the four wire lumen sets are arranged in four directions in the cross section of the base end side tubular body,
wherein the four wire lumens are arranged in four directions in the cross section of the distal end side tubular body, and arranged with the base end side tubular body and the distal end side tubular body that are arranged in series in a direction that the four directions are aligned,
wherein the long traction body of the traction body set is routed to the wire lumen in one of the four directions of the cross section in which the wire lumen is provided in the distal end side tubular body, and fixed to the distal end side tubular body at the distal end of the distal end side tubular body,
wherein the long and short traction bodies of the traction body set are arranged in the base end side tubular body in the wire lumen that is closer to the cross-sectional center of the tubular body in the one direction than the wire lumen arranged in the distal end side tubular body and faces the cross-sectional center in the one direction,
wherein the short traction body arranged in the wire lumen of the base end side tubular body is fixed to the base end side tubular body on the distal side of the base end side tubular body,
wherein each of the traction bodies is configured to be independently towable, and
wherein by towing the plurality of traction bodies toward the base end side, at least one of the base end side tubular body and the distal end side tubular body is curved and deformed in a desired direction relative to the other.

6. The movable elongated structure according to claim 2, wherein the first virtual line and the second virtual line pass through the cross-sectional center.

7. The movable elongated structure according to claim 6, in which the wiring aid is provided with a regulating part for regulating the relative positions of the traction body of one of the two traction body sets that cross between the tubular bodies adjacent in the longitudinal direction and the traction body of the other traction body set.

8. The movable elongated structure according to any one of claims 1 to 7, wherein the traction body is a flexible wire, and the wire lumen is a through path along the longitudinal direction provided inside the tube wall of the tubular body.

9. A movable elongated treatment instrument wherein a treatment instrument such as a retractor, gripper, forceps, tweezers, needle, probe, or scissors is provided at the distal end of the distal end side tubular body in the movable elongated structure according to claim 8, and a drive mechanism for the treatment instrument is arranged in the internal space of the tubular bodies.

10. The movable elongated structure according to claim 8, wherein the distal end side tubular body is an elastic retractor, elastic gripper, forceps or scissors having the internal space, and is opened and closed by the wire.

11. A method for inserting a movable elongated structure according to claim 10 into a duct, driving and controlling a traction drive unit that tows the traction body to bend and deform at least one of the distal end side tubular body and the base end side tubular body in a desired direction, and inserting the distal end side tubular body into a branching duct.

12. The method for inserting the movable elongated structure according to claim 11, in which the duct is at least one of a hollow organ, a vascular channel and a blood vessel.

13. A method for operating the movable elongated structure according to claim 8, in which the traction body set is towed to bend and deform at least one of the distal end side tubular body and the base end side tubular body in a desired direction.

14. A method for operating the movable elongated structure according to claim 5, in which a plurality of the traction body sets or one of the traction bodies in the plurality of the traction body sets is towed to bend and deform at least one of the distal end side tubular body and the base end side tubular body in a desired direction.

15. A method for operating the movable elongated structure according to any one of claims 1 to 3,
wherein one of the traction bodies in the traction body set is routed through the wire lumen of the base end side tubular body and the wire lumen of the distal end side tubular body, and is attached to the distal end side tubular body at the distal side of distal end side tubular body, and is towed to bend and deform the distal end side tubular body in a desired direction,
wherein the other of the traction body set is routed through the wire lumen of the base end tubular body and is attached to the base end side tubular body at the distal end side of the base end side tubular body, and is towed to bend and deform the base end tubular body in a desired direction.

16. A movable elongated structural instrument comprising the movable elongated structural body according to claim 10, and a traction drive unit for traction the traction body, wherein the traction drive unit tows the traction body to bend and deform at least one of the distal end side tubular body and the base end side tubular body in a desired direction.

17. A medical system comprising;
the movable elongated structural instrument according to claim 16,
a drive unit for driving the traction drive unit, and
a control unit connected to the drive unit to apply a drive signal to the same.

18. A tool comprising;
the movable elongated structural instrument according to claim 16,
an attachment unit for attaching the base end side tubular body of the movable elongated structural body to a distal end of a robot arm, and
a connection unit for connecting to a drive mechanism for driving the traction drive unit on the robot arm side.

19. A robot comprising;
the tool according to claim 18;
a robot arm having the tool at its distal end,
a drive unit for driving the traction drive section and the robot arm, and
a control unit connected to the drive unit to apply a drive signal to the same.

20. A robot comprising;
an input/output unit connected by wire and/or wirelessly to the movable elongated structural instrument according to claim 16,
an input unit for receiving an operation signal in real time,
a arithmetic unit for executing a predetermined operation program based on the operation signal, and
an output unit for generating a drive signal for traction a predetermined traction body with the traction drive section based on an output from the calculation unit, thereby bending and deforming at least one of the distal end side tubular body and the base end side tubular body in a desired direction.

21. A robot comprising;
the tool according to claim 18;
a robot arm having the tool at its distal end,
a drive unit for driving the traction drive and the robot arm,
a control unit connected to the drive unit to apply a drive signal to the same, in which the control unit is provided with artificial intelligence.

22. A robot comprising,
a movable elongated structural instrument according to claim 16,
a manipulator having a main body provided at the base end of the base end side tubular body of the movable elongated structural instrument, and an operating unit in the main body for operating the traction drive unit,
a robot arm having the tool at its distal end,
a drive unit for driving the operating unit and the robot arm; and
a control unit connected to the drive unit to apply a drive signal to the same, wherein the control unit is equipped with artificial intelligence.

23. A robot comprising;
an input/output unit connected by wire and/or wireless to the movable elongated structural instrument according to claim 16,
an input unit receiving an operation signal in real time,
an arithmetic unit executing a predetermined operation program based on the operation signal, and
and an output unit that generates a drive signal for towing a predetermined traction body using the traction drive unit based on an output from the arithmetic unit, thereby bending and deforming at least one of the distal end side tubular body and the base end side tubular body in a desired direction,
wherein the arithmetic unit is equipped with artificial intelligence.

24. A robot operation method comprising;
receiving an operation signal in real time by an input/output unit connected by wire and/or wireless to a robot equipped with the movable elongated structural instrument according to claim 16,
executing a predetermined operation program based on the received operation signal by an arithmetic unit,
whereby the traction body is towed by the traction drive unit based on the output from the arithmetic unit, thereby bending and deforming at least one of the distal end side tubular body and the base end side tubular body in a desired direction.

25. A medical robot comprising the robot according to claim 20,
wherein the output unit provides a drive signal to an external drive unit that mechanically drives the movable elongated structure.

26. A manipulator comprising;
the movable elongated structure instrument according to claim 16,
a main body provided at the base end of the base end side tubular body of the movable elongated structure, and
an operation unit in the main body that operates the traction drive unit.

27. A flexible endoscope comprising;
the movable elongated structure according to claim 10, and
a plurality of traction operation units that tows the traction bodies,
wherein the traction operation units tow the traction bodies to bend and deform at least one of the distal end side tubular body and the base end side tubular body in a desired direction.

28. A steering catheter comprising;
the movable elongated structure according to claim 10, and
a plurality of traction operating units for towing the traction bodies, and
wherein the traction body is towed by the traction operating units to bend and deform at least one of the distal end side tubular body and the base end side tubular body in a desired direction.

29. A movable elongated structure comprising;
a plurality of tubular bodies formed in a flexible, long shape and having an internal space passing through in the longitudinal direction,
a flexible, long-shaped traction body.
a wiring aid arranged between two of the tubular bodies arranged in series in the longitudinal direction and maintaining a distance between the opposing ends of the tubular bodies,
wherein the direction in which the traction body is towed in the longitudinal direction is the base end side and the opposite side is the distal end side,
wherein in the two tubular bodies adjacent in the longitudinal direction among the plurality of tubular bodies arranged in series, the tubular body arranged on the base end side is the base end side tubular body and the tubular body arranged on the distal end side is the distal end side tubular body,
wherein a wire lumen is provided in the tubular bodies for wiring the traction body along the longitudinal direction,
wherein a pair of the traction bodies is a traction body set, and a pair of the wire lumens is a wire lumens set,
wherein in the base end side tubular body, the wire lumens are arranged in a wire lumen that is closer to the cross-sectional center of the tubular body in the one direction and has a wider interval between the wire lumens than the wire lumen set arranged in the distal end side tubular body,
wherein in the base end side tubular body, a first virtual line connecting the traction bodies of one of the two sets of the traction body sets arranged in one of the wire lumens in the two sets of the wire lumen sets in the cross-sectional direction and a second virtual line connecting the traction bodies of the other set of the traction body in the cross-sectional direction intersect in the cross-sectional direction,
wherein by towing the plurality of traction bodies toward the base end side, at least one of the base end side tubular body and the distal end tubular body is curved and deformed in a desired direction relative to the other.
